# EUROPEAN PATENT SPECIFICATION

(11) **EP 1 565 425 B1**
(45) Date of publication and mention of the grant of the patent: **20.02.2008**
(21) Application number: 03769682.0
(22) Date of filing: 31.10.2003
(51) Int. Cl.: C07C 67/38, C07C 69/68

(54) **Production process of a lactate ester or acid**
Herstellungsverfahren von Laktat Ester- oder Säure
Procédé de production de l'ester de lactate ou de l'acide lactique

(30) Priority: 30.11.2002 GB 0228018
(43) Date of publication of application: 24.08.2005
(73) Proprietor: Lucite International UK Limited, Southampton, Hampshire SO14 3BP (GB)
(72) Inventor: EASTHAM, Graham Ronald, Co Durham DL1 5TQ (GB); RUCKLIDGE, Adam, John, Dundee DD1 5RA (GB); COLE-HAMILTON, David, St. Andrews, Fife KY16 9ST (GB)
(74) Representative: Walsh, David Patrick
(86) International application number: PCT/GB2003/004679
(87) International publication number: WO 2004/050599

(56) References cited:
- EP-A- 0 495 548
- WO-A-01/68583
- US-A- 4 377 708
- US-A- 4 500 727

## Description

The present invention relates to the carbonylation of an ester, specifically vinyl acetate and in particular the use of the carbonylation to provide a first step in the production of methyl lactate.

Currently methyl lactate is produced by esterification of lactic acid, which is produced either by synthetic methods or fermentation,

The main synthetic routes are based on the reactions of acetaldehyde. In one method, acetaldehyde is reacted with hydrogen cyanide to produce a lactonitrile, which is then hydrolysed. Alternatively, acetaldehyde can be reacted with carbon monoxide and water in the presence of a nickel (II) iodide or sulphuric acid catalyst. Synthetic routes produce racemic mixtures of lactic acid, and so racemic mixtures of methyl lactate result. In recent years, improvements in fermentation methods have made this: a preferred route to lactic acid and its derivatives. Optically pure lactic acid can be produced by the i fermentation of sugars with carefully chosen bacteria. Lactobacilli tend to be heat resistant so fermentation at temperatures of around 50°C suppresses secondary reactions. The procedure is slow, and requires careful monitoring of pH, temperature and oxygen levels, but by selecting an appropriate bacteria culture, optically pure lactic acid, of both R and S forms can be produced.

Methyl lactate is used as a high boiling point solvent, and is present in a variety of materials such as detergents, degreasing agents, cosmetics and food flavourings. It is biodegradable, and so environmentally friendly.

A route to 1, 3-propanediol would be industrially favourable, as there is currently not a route to the diol that is commercially viable. In the 1980s, Davy Process Technology found a route to 1, 4-butanediol, by forming diethyl maleate from butanes over a solid acid catalyst, and then dehydrogenating it to the diol. 1, 4-butanediol is now widely as a polymer component and also in fibre production and as a high boiling solvent. Polyhydric alcohols are often used in reactions with isocyanates to produce urethanes, and in reactions with acids and acid anhydrides to produce (poly) esters. 1, 3-propanediot is thought to have uses as a polymer component and as a high boiling point solvent.

The carbonylation of ethylenically unsaturated compounds using carbon monoxide in the presence of an alcohol or water and a catalyst system comprising a group VIII metal, example, palladium, and a phosphine ligand, example an alkyl phosphine, cycloalkyl phosphine, aryl phosphine, pyridyl phosphine or bidentate phosphine, has been described in numerous European patents and patent applications, example EP-A-0055875, EP-A-04489472, EP-A-0106379, EP-A-0235864, EP-A-0274795, EP-A-0499329, EP-A-0386833, EP-A-0441447, EP-A-0489472, EP-A-0282142, EP-A-0227160, EP-A-0495547 and EP-A-0495548. In particular, EP-A-0227160, EP-A-0495547 and EP-A-0495548 disclose that bidentate phosphine ligands provide catalyst systems which enable high reaction rates to be achieved.

The main problem with the previously disclosed catalyst systems is that, although relatively high reaction rates can be achieved, the palladium catalyst dies off quickly which is industrially unattractive.

It has been disclosed in WO96/19434 that a particular group of bidentate phosphine compounds can provide remarkably stable catalysts which require little or no replenishment; that use of such bidentate catalysts leads to reaction rates which are significantly higher than those previously disclosed; that little or no impurities are produced at high conversions.

In addition, WO 96/19434 discloses that the same catalyst process when used with respect to propene has been found to be more difficult.

WO 01/68583 discloses rates for the same process used for higher alkenes of C3 or more carbon atoms when in the presence of an externally added aprotic solvent.

EP0495548B1 gives an example of vinyl acetate carbonylation employing the C3 bridged phosphine 1,3bis (di-tert-butylphosphino) propane. The rates quoted are 200 moles product per mole of Pd per hour and the result is the production of 1 and 2 - acetoxy methyl propanoate in a ratio of 40:60 (linear:branched).

WO 01/68583 discloses a high regioselectivity for the linear product when carrying out carbonylation with a bidentate phosphine having an aryl bridge linked to the respective phosphines via adjacent carbons on the ring.

Production of lactate ester from vinyl acetate is known from US 4500727 via hydroformylation and oxidation.

However, surprisingly, it has been found that the carbonylation of vinyl acetate produces mainly branched product, rather than the linear product obtained with other substrates, with the same bidentate phosphine aryl bridged ligand.

According to a first aspect of the present invention there is provided a process for the production of a lactate ester or acid of formula II 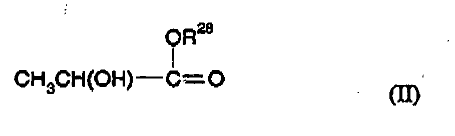 comprising the steps of carbonylating a vinyl acetate compound of formula

R²⁹-C(O)O CR³⁰ = CR³¹ R³²

wherein R²⁹ may be selected from hydrogen, C₁ - C₁₀ alkyl, aryl, Het, halo, cyano, nitro, OR¹⁹, OC(O)R²⁰, C(O)R²¹, C(O)OR²², NR²³R²⁴, C(O)NR²⁵R²⁶, C(S) R²⁵R²⁶, SR²⁷, C(O) SR²⁹ wherein R¹² R¹⁸ and R¹⁹-R²⁷ are as defined below and R³⁰-R³² represent hydrogen,
with carbon monoxide in the presence of a source of hydroxy groups and of a catalyst system, the catalyst system obtainable by combining:
(a) a metal of Group VIII B or a compound thereof; and
(b) a bidentate phosphine of general formula (I). 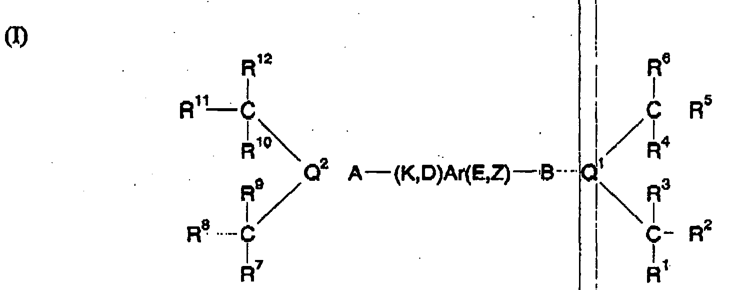
wherein:
Ar is a bridging group comprising an optionally substituted aryl moiety to which the phosphorus atoms are linked on available adjacent carbon atoms;
A and B each independently represent C₁-C₁₀ alkylene;
K, D, E and Z are substituents of the aryl moiety (Ar) and each independently represent hydrogen, C₁-C₁₀ alkyl, aryl, Het, halo, cyano, nitro, OR¹⁹, OC(O)R²⁰, C(O)R²¹, C(O)OR²²,
NR²³R²⁴, C(O)NR²⁵R²⁶, C(S)R²⁵R²⁶ SR²⁷, C(O)SR²⁷, or -J-Q³(CR¹³(R¹⁴)(R¹⁵))CR¹⁶(R¹⁷)(R¹⁸) where J represents C₁-C₁₀ alkylene; or two adjacent groups selected from K, Z, D and E together with the carbon atoms of the aryl ring to which they are attached form a further phenyl ring, which is optionally substituted by one or more substituents selected from hydrogen, C₁-C₁₀ alkyl, halo, cyano, nitro, OR¹⁹, OC(O)R²⁰, C(O)R²¹, C(O)OR²², NR²³R²⁴, hydrogen, alkyl, halo, cyano, nitro, OR¹⁹, OC(O)R²⁰, C(O)R²¹ C(O)OR²², C(O)NR²⁵R²⁶, C(S)R²⁵R²⁶, SR²⁷ or C(O)SR²⁷ or, when Ar is a cyclopentadienyl group, Z may be represented by -M(L₁)ₙ (L₂)ₘ and Z is connected via a metal ligand bond to the cyclopentadienyl group;
R¹ to R¹⁸ each independently represent C₁-C₁₀ alkyl, aryl, or Het;
R¹⁹ to R²⁷ each independently represent hydrogen, C₁-C₁₀ alkyl, aryl or Het;
M represents a Group VIB or VIIIB metal or metal cation thereof,
L₁ represents a cyclopentadienyl, indenyl or aryl group each of which groups are optionally substituted by one or more substituents selected from hydrogen C₁-C₁₀ alkyl, halo, cyano, nitro, OR¹⁹*,* OC(O)R²⁰, C(O)R²¹, C(O)OR²², NR²³R²⁴, C(O)NR²⁵R²⁶, C(S)R²⁶R²⁶, SR²⁷, C(O)SR²⁷or ferrocenyl;
L₂ represents one or more ligands each of which are independently selected from hydrogen, C₁-C₁₀ alkyl, alkylaryl, halo, CO, PR⁴³R⁴⁴R⁴⁵ or NR⁴⁶R⁴⁷R⁴⁸;
R⁴³ to R⁴⁸ each independently represent hydrogen, C₁-C₁₀ alkyl, aryl or Het;
n = 0 or 1 ;
and m = 0 to 5;
provided that when n = 1 then m equals 0, and when n equals, 01 then m does not equal 0;
Q¹, Q² and Q³ (when present) each independently represent phosphorous, arsenic or antimony and in the latter two cases references to phosphine or phosphorus above are amended accordingly,
to produce a product comprising a branched (iso) product and chemically treating said branched (iso) product to produce the corresponding lactate or and of formula II, wherein R²⁸ is selected from H, C₁-C₃₀ alkyl or aryl moiety which may be substitued or unsubstitued and either branched or linear. Preferably the Group VIII B metal is palladium.

By treating or treatment herein is meant carrying out routine chemical treatment such as hydrolysis or transesterification reactions on the branched (iso) product of the carbonylation suitable to produce the hydroxy acid or ester.

The linear (n) and branched (iso) products of the carbonylation may be separated either before or after the treatment step. Preferably, the products of the reaction are separated by distillation. The branched and linear products often have widely differing boiling points which makes distillation an effective separation technique for the products of the reaction.

Preferably, the ratio of branched :linear product from the carbonylation process is greater than 1.5:1, more preferably, greater than 2:1, most preferably greater than 2.5:1.

According to a further aspect of the present invention there is provided the use of the catalyst system as defined in claim 1 of the present invention for the production, preferably, industrial production, of a lactate ester or acid of formula (II) the said production comprising the steps of carbonylation of a vinyl acetate followed by treatment of the branched (iso) product of the carbonylation to produce the ester or acid.

Advantageously, the lactate esters or acids of the present invention may be hydrogenated to produce the 1,2 diols.

Preferably, the treatment is hydrolysis or transesterification and is carried out by any suitable technique known to those skilled in the art. Such techniques are detailed in for example - "Kirkothmer Encyclopaedia of Chemical Technology", volume 9, 4th edition page 783 - "Hydrolysis of Organic Esters". Such methods include base hydrolysis, acid hydrolysis, steam hydrolysis and enzymic hydrolysis. Preferably, the hydrolysis is base hydrolysis, more preferably, the hydrolysis is carried out in excess base and then acidified to produce the acid product. Hydrogenation of the hydrolysis product may be carried out by any suitable process known to those skilled in the art. Preferably, vapour phase hydrogenation of the hydroxy alkanoate ester is carried out. A suitable technique has been exemplified in WO 01170659 by Crabtree et al. Suitable experimental details are set out in examples 1-9 of the published application and illustrate the route to 1,3 propanediol from 3-hydroxy propanoic acid esters. Preferably, the hydrogenation is carried out in a hydrogenation zone containing a heterogenous hydrogenation catalyst. Suitable conditions and catalysts are set out in WO 01/70659 insofar as they relate to the hydrogenation of 3-hydroxy propanoic acid esters.

However, for the purposes of the present application such hydrogenation reactions are also deemed applicable to hydrogenation of the lactate ester to produce 1,2 propane diol.

Preferably, the transesterification is carried out with the alkanol corresponding to the alkyl group of the alkyl ester product required for example methanol for converting acetoxy alkyl esters into hydroxy methyl esters and ethanol for converting acetoxy alkyl esters into hydroxy ethyl esters etc. Advantageously, this cleaves the acetoxy group but does not alter the hydroxy alkyl alkanoate. Preferably, the transesterification takes place in the presence of a suitable catalyst such as for example methane sulphonic acid or p-toluene sulphonic acid.

Preferably, when K, D, E or Z represent -J-Q³(CR¹³(R¹⁴)(R¹⁵))CR¹⁶(R¹⁷)(R¹⁸), the respective K, D, E or Z is on the aryl carbon adjacent the aryl carbon to which A or B is connected or, if not so adjacent, is adjacent a remaining K, D, E or Z group which itself represents -J-Q³(CR¹³(R¹⁴)(R¹⁵))CR¹⁶(R¹⁷)(R¹⁸).

Suitably, the process of the invention may be used to catalyse the carbonylation of a vinyl acetate compound in the presence of carbon monoxide and a hydroxyl group containing compound as a first step i.e. the process of the invention may catalyse the conversion of a vinyl acetate compound to the corresponding acetoxy carboxylic acid or ester, respectively, depending on the choice of hydroxyl group containing compound used. Conveniently, the first step of the process of the invention may utilise highly stable compounds under typical carbonylation reaction conditions such that they require little or no replenishment. Conveniently, this first step may have a high rate for the carbonylation reaction of a vinyl acetate compound. Conveniently, the process of the invention may promote high conversion rates of the vinyl acetate compound, thereby yielding the branched (iso) product in high yield with little or no impurities.

The term "Ar" or "aryl" when used herein, includes five-to-ten-membered, preferably six to ten membered, carbocyclic aromatic or pseudo aromatic groups, such as phenyl, ferrocenyl and naphthyl, which groups are optionally substituted with, in addition to K, D, E or Z, one or more substituents selected from aryl, lower alkyl (which alkyl group may itself be optionally substituted or terminated as defined below), Het, halo, cyano, nitro, OR¹⁹, OC(O)R²⁰, C(O)R²¹ C(O)OR²² NR²³R²⁴, C(O)NR²⁵R²⁶, SR²⁷, C(O)SR²¹ or C(S)NR²⁵R²⁶ wherein R¹⁹ to R²⁷ each independently represent hydrogen, aryl or lower alkyl (which alkyl group may itself be optionally substituted or terminated as defined below).

Suitably, when Ar or aryl is cyclopentadienyl and when D and E together with the carbon atoms of the cyclopentadienyl ring to which they are attached form a phenyl ring, the metal M or cation thereof is attached to an indenyl ring system.

By the term "M represents a Group VIB or VIIIB metal" in a compound of formula I we include metals such as Cr, Mo, W, Fe, Co, Ni, Ru, Rh, Os, Ir, Pt and Pd. Preferably, the metals are selected from Cr, Mo, W, Fe, Co, Ni, Ru and Rh. For the avoidance of doubt, references to Group VIB or VIIIB metals herein should be taken to include Groups 6, 8, 9 and 10 in the modem periodic table nomenclature.

By the term "metal cation thereof' we mean that the Group VIB or VIIIB metal (M) in the compound of formula I as defined herein has a positive charge. Suitably, the metal cation may be in the form of a salt or may comprise weakly coordinated anions derived from halo, nitric acid; sulphuric acid; lower alkanoic (up to C₁₂) acids such as acetic acid and propionic acid; sulphonic acids such as methane sulphonic acid, chlorosulphonic acid, fluorosulphonic acid, trifluoromethane sulphonic acid, benzene sulphonic acid, naphthalene sulphonic acid, toluene sulphonic acid, e.g. p-toluene sulphonic acid, t-butyl sulphonic acid, and 2-hydroxypropane sulphonic acid; sulphonated ion exchange resins; perhalic acid such as perchloric acid; perfluororated carboxylic acid such as trichloroacetic acid and trifluoroacetic acid; orthophosphoric acid; phosphonic acid such as benzene phosphonic acid; and acids derived from interactions between Lewis acids and Broensted acids. Other sources which may provide suitable anions include the tetraphenyl borate derivatives.

Preferably M represents a Group VIB or VIIIB metal. In other words the total electron count for the metal M is 18.

Halo groups, which L₂ may represent and with which the above-mentioned groups may be substituted or terminated, include fluoro, chloro, bromo and iodo.

Suitably, if A represents cyclopentadienyl and n = 1, the compounds of formula I may contain either two cyclopentadienyl rings, two indenyl rings or one indenyl and one cyclopentadienyl ring (each of which ring systems may optionally be substituted as described herein). Such compounds may be referred to as "sandwich compounds" as the metal M or metal cation thereof is sandwiched by the two ring systems. The respective cyclopentadienyl and/or indenyl ring systems may be substantially coplanar with respect to each other or they may be tilted with respect to each other (commonly referred to as bent metallocenes).

Alternatively, when n = 1, the compounds of the invention may contain either one cyclopentadienyl or one indenyl ring (each of which ring systems may optionally be substituted as described herein) and one aryl ring (i.e. L₁ represents aryl) which is optionally substituted as defined herein. Suitably, when n = 1 and L₁ represents aryl then the metal M of the compounds of formula I as defined herein is typically in the form of the metal cation.

Suitably, when n = 0, the compounds of the invention contain only one cyclopentadienyl or indenyl ring (each of which ring systems may optionally be substituted as described herein). Such compounds may be referred to as "half sandwich compounds". Preferably, when n = 0 then m represents 1 to 5 so that the metal M of the compounds of formula I has an 18 electron count. In other words, when metal M of the compounds of formula I is iron, the total number of electrons contributed by the ligands L₂ is typically five.

Suitably, the metal M or metal cation thereof in the cyclopentadienyl compounds of formula I is typically bonded to the cyclopentadienyl ring(s) or the cyclopentadienyl moiety of the indenyl ring(s). Typically, the cyclopentadienyl ring or the cyclopentadienyl moiety of the indenyl ring exhibits a pentahapto bonding mode with the metal; however other bonding modes between the cyclopentadienyl ring or cyclopentadienyl moiety of the indenyl ring and the metal, such as trihapto coordination, are also embraced by the scope of the present invention.

Preferably, in the compound of formula I wherein Ar is cyclopentadienyl, M represents Cr, Mo, Fe, Co or Ru, or a metal cation thereof. Even more preferably, M represents Cr, Fe, Co or Ru or a metal cation thereof. Most preferably, M is selected from a Group VIIIB metal or metal cation thereof. An especially preferred Group VIIIB metal is Fe. Although the metal M as defined herein may be in a cationic form, preferably it carries essentially no residual charge due to coordination with L₁ and/or L₂ as defined herein.

Preferably, when n = I in the compound of formula I, L₁ represents cyclopentadienyl, indenyl or aryl each of which rings are optionally substituted by one or more substituents selected from hydrogen, lower alkyl, halo, cyano, OR¹⁹, OC(O)R²⁰, C(O)R²¹, C(O)OR²², NR²³R²⁴, SR²⁷ or ferrocenyl (by which is meant the cyclopentadienyl, indenyl or aryl ring which L₁ may represent is bonded directly to the cyclopentadienyl ring of the metallocenyl group). More preferably, if the cyclopentadienyl, indenyl or aryl ring which L₁ may represent is substituted it is preferably substituted with one or more substituents selected from C₁-C₆ alkyl, halo, cyano, OR¹⁹, OC(O)R²⁰, C(O)R²¹, C(O)OR²², NR²³R²⁴ where R¹⁹, R²⁰, R²¹, R²², R²³ and R²⁴ each independently represent hydrogen or C₁-C₆ alkyl.

Preferably, when n = 1, L₁ represents cyclopentadienyl, indenyl, phenyl or naphthyl optionally substituted as defined herein. Preferably, the cyclopentadienyl, indenyl, phenyl or naphthyl groups are unsubstituted. More preferably, L₁ represents cyclopentadienyl, indenyl or phenyl, each of which rings are unsubstituted. Most preferably, L₁ represents unsubstituted cyclopentadienyl.

In a particularly preferred embodiment of the present invention, in a compound of formula I, n = 1, L₁ is as defined herein and m = 0.

Alternatively, when n is equal to zero and m is not equal to zero in a compound of formula I, L₂ represents one or more ligands each of which are independently selected from lower alkyl, halo, CO, PR⁴³ R⁴⁴ R⁴⁵ or NR⁴⁶R⁴⁷ R⁴⁸. More preferably, L₂ represents one or more ligands each of which are independently selected from C₁ to C₄ alkyl, halo, particularly chloro, CO, PR⁴³R⁴⁴R⁴⁵ or NR⁴⁶R⁴⁷R⁴⁸, wherein R⁴³ to R⁴⁸ are independently selected from hydrogen, C₁ to C₆ alkyl or aryl, such as phenyl.

In a particularly preferred alternative embodiment of the present invention, in a compound of formula I, n = 0, L₂ is as defined herein and m = 3 or 4, particularly 3.

M represents a metal selected from Cr, Mo, Fe, Co or Ru or a metal cation thereof;
L₁ represents cyclopentadienyl, indenyl, naphthyl or phenyl, each of which rings may be optionally substituted by one or more substituents selected from C₁-C₆ alkyl, halo, cyano, OR¹⁹, OC(O)R²⁰, C(O)R²¹, C(O)R²², NR²³R²⁴;
L₂ represents one or more ligands each of which ligands are independently selected from C₁-C₆ alkyl, halo, CO, PR⁴³R⁴⁴R⁴⁵ or NR⁴⁶R⁴⁷R⁴⁸;
n = 0 or 1;
and m = 0 to 4;
provided that when n = 1 then m = 0 and when m does not equal zero then n = 0.

Further preferred compounds of formula I include those wherein:
M represents iron or a cation thereof;
L₁ represents cyclopentadienyl, indenyl or phenyl group, each of which groups are optionally substituted by one or more substituents selected from C₁-C₆ alkyl, halo, cyano, OR¹⁹, OC(O)R²⁰, C(O)R²¹, C(O)R²²;
L₂ represents one or more ligands each of which are independently selected from C₁-C₆ alkyl, halo, CO, PR⁴³R⁴⁴R⁴⁵ or N R⁴⁶ R⁴⁷ R ⁴⁸ where R⁴³ to R⁴⁸ are independently selected from hydrogen, C₁-C₆ alkyl or phenyl;
n = 0 or 1; and m = 0 to 4.

Still further preferred compounds of formula I include those wherein:
L₁ represents unsubstituted cyclopentadienyl, indenyl or phenyl, particularly unsubstituted cyclopentadienyl; and, n = 1 and m = 0.

Alternative preferred compounds of formula I include those wherein:
n=0;
L₂ represents one or more ligands each of which are independently selected from C₁ to C₆ alkyl, halo, CO, PR⁴³R⁴⁴R⁴⁵ or NR⁴⁶R⁴⁷R⁴⁸, where R⁴³ to R⁴⁸ are independently selected from hydrogen, C₁-C₆ alkyl or phenyl; and m = 1 to 4, particularly 3 or 4. For example, when m=3 the three ligands which L₂ may represent include (CO)₂halo, (PR⁴³R⁴⁴R⁴⁵)₂halo or (NR⁴⁶R⁴⁷R⁴⁸)₂halo.

References to vinyl acetate herein include references to substituted or unsubstituted vinyl acetate of formula (IV):

R²⁹ - C(O) O CR³⁰ = CR³¹ R³²

wherein R²⁹ may be selected from hydrogen, lower alkyl, aryl, Het, halo, cyano, nitro, OR¹⁹, OC(O)R²⁰, C(O)R²¹, C(O)OR²², NR²³R²⁴, C(O)NR²⁵R²⁶ , C(S) R²⁵R²⁶, SR²⁷, C(O) SR²⁹ wherein R¹² -R¹⁸ and R¹⁹-R²⁷ are as defined herein.

Preferably, R²⁹ is selected from hydrogen, lower alkyl, phenyl or lower alkylphenyl. More preferably, hydrogen, phenyl, C₁-C₆ alkylphenyl or C₁-C₆ alkyl, such as methyl, ethyl, propyl, butyl, pentyl and hexyl, even more preferably, C₁-C₆ alkyl, especially methyl.

R³⁰-R³² represent hydrogen. As mentioned above, R²⁸ may be optionally substituted, preferably, with one or more substituents selected from lower alkyl, aryl, Het, halo, cyano, nitro, OR¹⁹, OC(O)R²⁰, C(O)R²¹, C(O)OR²², NR²³R²⁴, C(O)NR²⁵SR²⁶, C(S)R²⁵R²⁶, SR²⁷ or C(O)SR²⁷ as defined herein.

R²⁸ is most preferably the alkyl/aryl group derived from a C₁-C₈ alkanol such as methanol, ethanol, propanol, iso-propanol, iso-butanol, t-butyl alcohol, n-butanol, phenol and chlorocapryl alcohol. The most preferred groups are methyl and ethyl, the most especially preferred group is methyl.

The term "Het", when used herein, includes four-to-twelve-membered, preferably four-to-ten-membered ring systems, which rings contain one or more heteroatoms selected from nitrogen, oxygen, sulphur and mixtures thereof, and which rings may contain one or more double bonds or be non-aromatic, partly aromatic or wholly aromatic in character. The ring systems may be monocyclic, bicyclic or fused. Each "Het" group identified herein is optionally substituted by one or more substituents selected from halo, cyano, nitro, oxo, lower alkyl (which alkyl group may itself be optionally substituted or terminated as defined below) OR¹⁹, OC(O)R²⁰, C(O)R²¹, C(O)OR²², NR²³R²⁴, C(O)NR²⁵R²⁶, SR²⁷, C(O)SR²⁷ or C(S)NR²⁵R²⁶ wherein R¹⁹ to R²⁷ each independently represent hydrogen, aryl or lower alkyl (which alkyl group itself may be optionally substituted or terminated as defined below). The term "Het" thus includes groups such as optionally substituted azetidinyl, pyrrolidinyl, imidazolyl, indolyl, furanyl, oxazolyl, isoxazolyl, oxadiazolyl, thiazolyl, thiadiazolyl, triazolyl, oxatriazolyl, thiatriazolyl, pyridazinyl, morpholinyl, pyrimidinyl, pyrazinyl, quinolinyl, isoquinolinyl, piperidinyl, pyrazolyl and piperazinyl. Substitution at Het may be at a carbon atom of the Het ring or, where appropriate, at one or more of the heteroatoms.

"Het" groups may also be in the form of an N oxide.

The term "lower alkyl" when used herein, means C₁ to C₁₀ alkyl and includes methyl, ethyl, propyl, butyl, pentyl, hexyl and heptyl groups. Unless otherwise specified, alkyl groups may, when there is a sufficient number of carbon atoms, be linear or branched, be saturated or unsaturated, be cyclic, acyclic or part cyclic/acyclic, and/or be substituted or terminated by one or more substituents selected from halo, cyano, nitro, OR¹⁹, OC(O)R²⁰, C(O)R²¹, C(O)OR²², NR²³R²⁴, C(O)NR²⁵R²⁶, SR²⁷, C(O)SR²⁷, C(S)NR²⁵R²⁶, aryl or Het, wherein R¹⁹ to R²⁷ each independently represent hydrogen, aryl or lower alkyl, and/or be interrupted by one or more oxygen or sulphur atoms, or by silano or dialkylsilcon groups.

Lower alkyl groups or alkyl groups which R¹, R², R³, R⁴, R⁵, R⁶, R⁷, R⁸, R⁹, R¹⁰, R¹¹, R¹², R¹³, R¹⁴, R¹⁵ , R¹⁶ , R¹⁷, R¹⁸ R¹⁹ R²⁰ , R²¹ , R²² , R²³ , R²⁴ , R²⁵ , R²⁶, R²⁷, R²⁸ , R²⁹ , R³⁰, R³¹, R³² K, D, E and Z may represent and with which aryl and Het may be substituted, may, when there is a sufficient number of carbon atoms, be linear or branched, be saturated or unsaturated, be cyclic, acyclic or part cyclic/acyclic, and/or be interrupted by one or more of oxygen or sulphur atoms, or by silano or dialkylsilicon groups, and/or be substituted by one or more substituents selected from halo, cyano, nitro, OR¹⁹, OC(O)R²⁰, C(O)R²¹, C(O)OR²², NR²³R²⁴, C(O)NR²⁵R²⁶, SR²⁷, C(O)SR²⁷, C(S)NR²⁵R²⁶, aryl or Het wherein R¹⁹ to R²⁷ each independently represent hydrogen, aryl or lower alkyl.

Similarly, the term "lower alkylene" which A, B and J (when present) represent in a compound of formula I, when used herein, includes C₁ to C₁₀ groups which can be bonded at two places on the group and is otherwise defined in the same way as "lower alkyl".

Halo groups with which the above-mentioned groups may be substituted or terminated include fluoro, chloro, bromo and iodo.

Where a compound of a formula herein (eg. formulas I-IV) contains an alkenyl group, cis (E) and trans (Z) isomerism may also occur. The present invention includes the individual stereoisomers of the compounds of any of the formulas defined herein and, where appropriate, the individual tautomeric forms thereof, together with mixtures thereof. Separation of diastereoisomers or cis and trans isomers may be achieved by conventional techniques, e.g. by fractional crystallisation, chromatography or H.P.L.C. of a stereoisomeric mixture of a compound one of the formulas or a suitable salt or derivative thereof. An individual enantiomer of a compound of one of the formulas may also be prepared from a corresponding optically pure intermediate or by resolution, such as by H.P.L.C. of the corresponding racemate using a suitable chiral support or by fractional crystallisation of the diastereoisomeric salts formed by reaction of the corresponding racemate with a suitable optically active acid or base, as appropriate.

All stereoisomers are included within the scope of the process of the invention.

It will be appreciated by those skilled in the art that the compounds of formula I (b) may function as ligands that coordinate with the Group VIIIB metal or compound thereof (a) to form the compounds for use in the invention. Typically, the Group VIIIB metal or compound thereof (a) coordinates to the one or more phosphorous, arsenic and/or antimony atoms of the compound of formula I.

Preferably, R¹ to R¹⁸ and R²⁸ each independently represent lower alkyl or aryl. More preferably, R¹ to R¹⁸ and R²⁸ each independently represent C₁ to C₆ alkyl, C₁-C₆ alkyl phenyl (wherein the phenyl group is optionally substituted as defined herein) or phenyl (wherein the phenyl group is optionally substituted as defined herein). Even more preferably, R¹ to R¹⁸ or R²⁸ each independently represent C₁ to C₆ alkyl, which is optionally substituted as defined herein. Most preferably, R¹ to R¹⁸ or R²⁸ each represent non-substituted C₁ to C₆ alkyl such as methyl, ethyl, n-propyl, iso-propyl, n-butyl, iso-butyl, tert-butyl, pentyl, hexyl and cyclohexyl.

Alternatively, or additionally, each of the groups R¹ to R³, R⁴ to R⁶, R⁷ to R⁹, R¹⁰ to R¹², R¹³ to R¹⁵ or R¹⁶ to R¹⁸ together independently may form cyclic structures such as 1-norbomyl, 1-norbornadienyl or adamantyl. Further examples of composite groups include cyclic structures formed between R¹ - R⁶ and R⁷ - R¹². Alternatively, one or more of the groups may represent a solid phase to which the ligand is attached.

In a particularly preferred embodiment of the present invention R¹, R⁴, R⁷, R¹⁰, R¹³ and R¹⁶ each represent the same lower alkyl, aryl or Het moiety as defined herein, R², R⁵, R⁸, R¹¹ , R¹⁴ and R¹⁷ each represent the same lower alkyl, aryl or Het moiety as defined herein, and R³, R⁶, R⁹, R¹², R¹⁵ and R¹⁸ each represent the same lower alkyl, aryl or Het moiety as defined herein. More preferably R¹, R⁴, R⁷, R¹⁰, R¹³ and R¹⁶ each represent the same C₁-C₆ alkyl, particularly non-substituted C₁-C₆ alkyl, such as methyl, ethyl, n-propyl, iso-propyl, n-butyl, iso-butyl, tert-butyl, pentyl, hexyl or cyclohexyl; R², R⁵, R⁸, R¹¹, R¹⁴ and R¹⁷ each independently represent the same C₁-C₆ alkyl as defined above; and R³, R⁶, R⁹, R¹², R¹⁵ and R¹⁸ each independently represent the same C₁-C₆ alkyl as defined above. For example: R¹, R⁴, R⁷, R¹⁰, R¹³ and R¹⁶ each represent methyl; R², R⁵, R⁸, R¹¹, R¹⁴ and R¹⁷ each represent ethyl; and, R³, R⁶, R⁹, R¹², R¹⁵ and R¹⁸ each represent n-butyl or n-pentyl.

In an especially preferred embodiment of the present invention each R¹ to R¹⁸ and R²⁸ group represents the same lower alkyl, aryl, or Het moiety as defined herein. Preferably, each R¹ to R¹⁸ represents the same C₁ to C₆ alkyl group, particularly non-substituted C₁-C₆ alkyl, such as methyl, ethyl, n-propyl, iso-propyl, n-butyl, iso-butyl, tert-butyl, pentyl, hexyl and cyclohexyl. Most preferably, each R¹ to R¹⁸ and R²⁸ represents methyl.

In the compound of formula I, preferably each Q¹, Q² and Q³ (when present) are the same. Most preferably, each Q¹, Q² and Q³ (when present) represents phosphorous.

Preferably, in the compound of formula I, A, B and J (when present) each independently represent C₁ to C₆ alkylene which is optionally substituted as defined herein, for example with lower alkyl groups. Preferably, the lower alkylene groups which A, B and J (when present) represent are non-substituted. A particular preferred lower alkylene which A, B and J may independently represent is -CH₂- or -C₂H₄-. Most preferably, each of A, B and J (when present) represent the same lower alkylene as defined herein, particularly -CH₂-.

Preferably, in the compound of formula I when K, D, E or Z does not represent -J-Q³(CR¹³(R¹⁴)(R¹⁵))CR¹⁶(R¹⁷)(R¹⁸), K, D, E or Z represents hydrogen, lower alkyl, phenyl or lower alkylphenyl. More preferably, K, D, E or Z represent hydrogen, phenyl, C₁-C₆ alkylphenyl or C₁-C₆ alkyl, such as methyl, ethyl, propyl, butyl, pentyl and hexyl. Most preferably, K, D, E or Z represents hydrogen.

Preferably, in the compound of formula I when K, D, E and Z together with the carbon atoms of the aryl ring to which they are attached do not form a phenyl ring, K, D, E and Z each independently represent hydrogen, lower alkyl, phenyl or lower alkylphenyl. More preferably, K, D, E and Z each independently represent hydrogen, phenyl, C₁-C₆ alkylphenyl or C₁-C₆ alkyl, such as methyl, ethyl, propyl, butyl, pentyl and hexyl. Even more preferably, K, D, E and Z represent the same substituent. Most preferably, they represent hydrogen.

Preferably, in the compound of formula I when K, D, E or Z docs not represent -J-Q³(CR¹³(R¹⁴)(R¹⁵))CR¹⁶(R¹⁷)(R¹⁸) and K, D, E and Z together with the carbon atoms of the aryl ring to which they are attached do not form a phenyl ring, each of K, D, E and Z represent the same group selected from hydrogen, lower alkyl, aryl, or Het as defined herein; particularly hydrogen or C₁-C₆ alkyl (more particularly unsubstituted C₁-C₆ alkyl), especially hydrogen.

Preferably, in the compound of formula I when two of K, D, E and Z together with the carbon atoms of the aryl ring to which they are attached form a phenyl ring, then the phenyl ring is optionally substituted with one or more substituents selected from aryl, lower alkyl (which alkyl group may itself be optionally substituted or terminated as defined below), Het, halo, cyano, nitro, OR¹⁹, OC(O)R²⁰, C(O)R²¹, C(O)OR²², NR²³R²⁴, C(O)NR²⁵R²⁶, SR²⁷, C(O)SR²⁷ or C(S)NR²⁵R²⁶ wherein R¹⁹ to R²⁷ each independently represent hydrogen or lower alkyl (which alkyl group may itself be optionally substituted or terminated as defined herein). More preferably, the phenyl ring is not substituted by any substituents i.e. it bears hydrogen atoms only.

Preferred compounds of formula I include those wherein:
A and B each independently represent unsubstituted C₁ to C₆ alkylene;
K, D, Z and E each independently represent hydrogen, C₁-C₆ alkyl, phenyl, C₁-C₆ alkylphenyl or -J-Q³(CR¹³(R¹⁴)(R¹⁵))CR¹⁶(R¹⁷)(R¹⁸) where J represents unsubstituted C₁ to C₆ alkylene; or two of K, D, Z and E together with the carbon atoms of the aryl ring to which they are attached form a phenyl ring which is optionally substituted by one or more substituents selected from lower alkyl, phenyl or lower alkylphenyl.
R¹ to R¹⁸ each independently represent C₁ to C₆ alkyl, phenyl or C₁ to C₆ alkylphenyl.

Further preferred compounds of formula I include those wherein:
A and B both represent -CH₂- or C₂H₄, particularly CH₂;
K, D, Z and E each independently represent hydrogen, C₁-C₆ alkyl phenyl or C₁-C₆ alkyl or -J-Q³(CR¹³(R¹⁴)(R¹⁵))CR¹⁶(R¹⁷)(R¹⁸) where J is the same as A; or two of K, D, E and Z together with the carbon atoms of the aryl ring to which they are attached form an unsubstituted phenyl ring;
R¹ to R¹⁸ each independently represent C₁ to C₆ alkyl;

Still further preferred compounds of formula I include those wherein:
R¹ to R¹⁸ are the same and each represents C₁ to C₆ alkyl, particularly methyl.

Still further preferred compounds of formula I include those wherein:
K, D, Z and E are each independently selected from the group consisting of hydrogen or C₁ to C₆ alkyl, particularly where each of K, D, Z and E represent the same group, especially where each of K, D, Z and E represent hydrogen; or
K represents -CH₂-Q³(CR¹³(R¹⁴)(R¹⁵))CR¹⁶(R¹⁷)(R¹⁸) and D, Z and E are each independently selected from the group consisting of hydrogen or C₁ to C₆ alkyl, particularly where both D and E represent the same group, especially where D, Z and E represent hydrogen.

Especially preferred specific compounds of formula I include those wherein:
each R¹ to R¹² is the same and represents methyl;
A and B are the same and represent -CH₂-;
K, D, Z and E are the same and represent hydrogen.

The present invention provides process for the production of lactate ester or acid of formula II comprising carbonylating a vinyl acetate compound by contacting a vinyl acetate compound with carbon monoxide and a hydroxyl group containing compound in the presence of a catalyst compound as defined in the present invention.

Suitably, the hydroxyl group containing compound includes water or an organic molecule having a hydroxyl functional group. Preferably, the organic molecule having a hydroxyl functional group may be branched or linear, and comprises an alkanol, particularly a C₁-C₃₀ alkanol, including aryl alkanols, which may be optionally substituted with one or more substituents selected from lower alkyl, aryl, Het, halo, cyano, nitro, OR¹⁹, OC(O)R²⁰, C(O)R²¹, C(O)OR²², NR²¹R²¹, C(O)NR²⁵R²⁶, C(S)R²⁵R²⁶, SR²⁷ or C(O)SR²⁷ as defined herein. Highly preferred alkanols are C₁-C₈ alkanols such as methanol, ethanol, propanol, iso-propanol, iso-butanol, t-butyl alcohol, n-butanol, phenol and chlorocapryl alcohol. Although the monoalkanols are most preferred, poly-alkanols, preferably, selected from di-octa ols such as diols, triols, tetra-ols and sugars may also be utilised. Typically, such polyalkanols are selected from 1, 2-ethanediol, 1,3-propanediol, glycerol, 1,2,4 butanetriol, 2-(hydroxymethyl)-1,3-propanediol, 1,2,6 trihydroxyhexane, pentaerythritol, 1,1,1 tri(hydroxymethyl)ethane, nannose, sorbase, galactose and other sugars. Preferred sugars include sucrose, fructose and glucose. Especially preferred alkanols are methanol and ethanol. The most preferred alkanol is methanol.

The amount of alcohol is not critical. Generally, amounts are used in excess of the amount of vinyl acetate compound to be carbonylated. Thus the alcohol may serve as the reaction solvent as well, although, if desired, separate solvents may also be used.

It will be appreciated that the end product of the reaction is determined at least in part by the source of hydroxyl group containing compound used. If water is used as the hydroxyl group containing compound then the end product is the corresponding carboxylic acid, whereas use of an alkanol produces the corresponding ester. Use of methanol conveniently produces the 2-acetoxy methyl propanoate or 3-acetoxymethyl propanoate.

In the process according to the present invention, the carbon monoxide may be used in pure form or diluted with an inert gas such as nitrogen, carbon dioxide or a noble gas such as argon. Small amounts of hydrogen, typically less than 5% by volume, may also be present.

The ratio (volume/volume) of vinyl acetate compound to hydroxyl group containing compound may vary between wide limits and suitably lies in the range of 1:0.1 to 1:10, preferably from between 2:1 to 1:2 and up to a large excess of hydroxyl group containing compounds when the latter is also the reaction solvent such as up to a 50:1 excess of hydroxyl group containing compounds.

The amount of the catalyst of the invention used in the carbonylation step of the vinyl acetate compound is not critical. Good results may be obtained when, preferably, the amount of Group VIII metal is in the range 10⁻⁷ to 10⁻¹ moles per mole of vinyl acetate compound, more preferably, 10⁻⁶ to 10⁻² moles, most preferably 10⁻⁵ to 10⁻² moles per mole of ethylenically unsaturated compound. Preferably, the amount of bidentate compound of formula I to unsaturated compound is in the range 10⁻⁷ to 10⁻¹, more preferably, 10⁻⁶ to 10⁻², most preferably, 10⁻⁵ to 10⁻² moles per mole of vinyl acetate compound.

Suitably, although non-essential to the invention, the carbonylation of a vinyl acetate compound as defined herein may be performed in one or more aprotic solvents. Suitable solvents include ketones, such as for example methylbutylketone; ethers, such as for example anisole (methyl phenyl ether), 2,5,8-trioxanonane (diglyme), diethyl ether, dimethyl ether, tetrahydrofuran, diphenylether, diisopropylether and the dimethylether of di-ethylene-glycol; esters, such as for example methylacetate, dimethyladipate methyl benzoate, dimethyl phthalate and butyrolactone; amides, such as for example dimethylacetamide, N-methylpyrrolidone and dimethyl formamide; sulfoxides and sulphones, such as for example dimethylsulphoxide, di-isopropylsulphone, sulfolane (tetrahydrothiophene-2,2-dioxide), 2-methylsulfolane, diethyl sulphone, tetrahydrothiophene 1,1-dioxide and 2-methyl-4-ethylsulfolane; aromatic compounds, including halo variants of such compounds eg. benzene, toluene, ethyl benzene o-xylene, m-xylene, p-xylene, chlorobenzene, o-dichlorobenzene, m-dichlorobenzene: alkanes, including halo variants of such compounds eg, hexane, heptane, 2,2,3-trimethylpentane, methylene chloride and carbon tetrachloride; nitriles eg. benzonitrile and acetonitrile.

Very suitable are aprotic solvents having a dielectric constant that is below a value of 50, more preferably in the range of 3 to 8, at 298.15 K and 1 x 10⁵Nm². In the present context, the dielectric constant for a given solvent is used in its normal meaning of representing the ratio of the capacity of a condenser with that substance as dielectric to the capacity of the same condenser with a vacuum for dielectric. Values for the dielectric constants of common organic liquids can be found in general reference books, such as the Handbook of Chemistry and Physics, 76^{th} edition, edited by David R. Lide et al, and published by CRC press in 1995, and are usually quoted for a temperature of about 20°C or 25°C, i.e. about 293.15k or 298.15 K, and atmospheric pressure, i.e. about 1 x 10⁵Nm⁻², or can readily be converted to that temperature and pressure using the conversion factors quoted. If no literature data for a particular compound is available, the dielectric constant may be readily measured using established physico-chemical methods.

For example, the dielectric constant of anisole is 4.3 (at 294.2 K), of diethyl ether is 4.3 (at 293.2 K), of sulfolane is 43.4 (at 303.2 K), of methylpentanoate is 5.0 (at 293.2 K), of diphenylether is 3.7 (at 283.2 K), of dimethyladipate is 6.8 (at 293.2 K), of tetrahydrofuran is 7.5 (at 295.2 K), of methylnonanoate is 3.9 (at 293.2 K). A preferred solvent is anisole.

If the hydroxyl group containing compound is an alkanol, an aprotic solvent will be generated by the reaction as the ester carbonylation product of the vinyl acetate compound, carbon monoxide and the alkanol is an aprotic solvent.

The process may be carried out in an excess of aprotic solvent, i.e. at a ratio (v/v) of aprotic solvent to hydroxyl group containing compound of at least 1:1. Preferably, this ratio ranges from 1:1 to 10:1 and more preferably from 1:1 to 5:1. Most preferably the ratio (v/v) ranges from 1.5:1 to 3:1.

Despite the aforegoing it is preferred that the reaction is carried out in the absence of any external added aprotic solvent ie. an aprotic solvent not generated by the reaction itself.

The catalyst compounds of the present invention may act as a "heterogeneous" catalyst or a "homogeneous" catalyst.

By the term "homogeneous" catalyst we mean a catalyst, i.e. a compound of the invention, which is not supported but is simply admixed or formed in-situ with the reactants of the carbonylation reaction (e.g. the vinyl acetate compound, the hydroxyl containing compound and carbon monoxide), preferably in a suitable solvent as described herein.

By the term "heterogeneous" catalyst we mean a catalyst, i.e. the compound of the invention, which is carried on a support.

The process of the present invention is carried out with the catalyst comprising a support, preferably an insoluble support.

Preferably, the support comprises a polymer such as a polyolefin, polystyrene or polystyrene copolymer such as a divinylbenzene copolymer or other suitable polymers or copolymers known to those skilled in the art; a silicon derivative such as a functionalised silica, a silicone or a silicone rubber; or other porous particulate material such as for example inorganic oxides and inorganic chlorides.

Preferably the support material is porous silica which has a surface area in the range of from 10 to 700 m²/g, a total pore volume in the range of from 0.1 to 4.0 cc/g and an average particle size in the range of from 10 to 500µm. More preferably, the surface area is in the range of from 50 to 500 m²/g, the pore volume is in the range of from 0.5 to 2.5 cc/g and the average particle size is in the range of from 20 to 200 µm. Most desirably the surface area is in the range of from 100 to 400 m²/g, the pore volume is in the range of from 0.8 to 3.0 cc/g and the average particle size is in the range of from 30 to 100 µm. The average pore size of typical porous support materials is in the range of from 10 to 1000 Å. Preferably, a support material is used that has an average pore diameter of from 50 to 500 Å, and most desirably from 75 to 350 Å. It may be particularly desirable to dehydrate the silica at a temperature of from 100°C to 800°C anywhere from 3 to 24 hours.

Suitably, the support may be flexible or a rigid support, the insoluble support is coated and/or impregnated with the compounds of the process of the invention by techniques well known to those skilled in the art.

Alternatively, the compounds of the process of the invention are fixed to the surface of an insoluble support, optionally via a covalent bond, and the arrangement optionally includes a bifunctional spacer molecule to space the compound from the insoluble support.

The compounds of the invention may be fixed to the surface of the insoluble support by promoting reaction of a functional group present in the compound of formula I, for example a substituent K, D, Z and E of the aryl moiety, with a complimentary reactive group present on or previously inserted into the support. The combination of the reactive group of the support with a complimentary substituent of the compound of the invention provides a heterogeneous catalyst where the compound of the invention and the support are linked via a linkage such as an ether, ester, amide, amine, urea, keto group.

The choice of reaction conditions to link a compound of the process of the present invention to the support depend upon the vinyl acetate compound and the groups of the support. For example, reagents such as carbodiimides, 1,1 -carbonyldiimidazole, and processes such as the use of mixed anhydrides, reductive amination may be employed.

The present invention provides the use of the process of the invention wherein the catalyst is attached to a support.

Particularly preferred is when the organic groups R¹, R², R³, R⁴, R⁵, R⁶, R⁷, R⁸, R⁹, R¹⁰, R¹¹ and R¹² or R¹ - R¹⁸ when associated with their respective carbon atom form composite groups which are at least as sterically hindering as t-butyl. Steric hindrance in this context is as discussed at page 14 et seq of "Homogenous Transition Metal Catalysis - A Gentle Art", by C Masters, published by Chapman and Hall 1981. These steric groups may be cyclic, part-cyclic or acyclic. When cyclic or part cyclic, the group may be substituted or unsubstituted or be saturated or unsaturated. The cyclic or part cyclic groups may contain, including the tertiary carbon atom, from C₄-C₃₀, more preferably C₆-C₂₀, most preferably C₁₀-C₁₅ carbon atoms in the cyclic structure. The cyclic structure may be substituted by one or more substituents selected from halo, cyano, nitro, OR¹⁹, OC(O)R²⁰, C(O)R²¹, C(O)OR²², NR²³R²⁴, C(O)NR²⁵R²⁶, SR²⁷, C(O)SR²⁷, C(S)NR²⁵R²⁶, aryl or Het, wherein R¹⁹ to R²⁷ each independently represent hydrogen, aryl or lower alkyl, and/or be interrupted by one or more oxygen or sulphur atoms, or by silano or dialkylsilcon groups.

The bridging group Ar is an aryl moiety, eg. a phenyl group, which may be optionally substituted, provided that the two phosphorus atoms are linked to adjacent carbon atoms, eg. at the 1 and 2 positions on the phenyl group. Furthermore, the aryl moiety may be a fused polycyclic group eg. naphthalene, biphenylene or indene.

Examples of suitable bidentate ligands are bis (di-t-butyl phosphino)-o-xylene (also known as 1,2 bis (di-t-butylphosphinomethyl) benzene); 1,2 bis (diadamantylphosphinomethyl) benzene; 1,2 bis (diadamantylphosphinomethyl) naphthalene; 1,2 bis (di-t-pentyl phosphino)-o-xylene (also known as 1,2 bis (di-t-pentyl-phosphinomethyl) benzene); and bis 2,3 (di-t-butyl phosphinomethyl) naphthalene. Additionally, the bidentate phosphine may be bonded to a suitable polymeric substrate via at least one of the bridging group Ar, the linking group A or the linking group B eg. bis (di-t-butyl phosphino)-o-xylene may be bonded via the xylene group to polystyrene to give an immobile heterogeneous catalyst. Examples of suitable bidentate ferrocene ligands are
1,2-bis-(ditertbutylphosphinomethyl) ferrocene;
1,2,3-tris-(ditcrtbutylphosphinomethyl) ferrocene;
1,2 bis (diadamantylphosphinomethyl) ferrocene; and
1,2 bis (di-t-pentyl phosphinomethyl) ferrocene.

The amount of bidentate ligand used can vary within wide limits. Preferably, the bidentate ligand is present in an amount such that the ratio of the number of moles of the bidentate ligand present to the number of moles of the Group VIII metal present is from 1 to 50 eg. 1 to 10 and particularly from 1 to 5 mol per mol of metal. More preferably, the mol:mol range of compounds of formula I to Group VIIIB metal is in the range of 1:1 to 3:1, most preferably in the range of 1:1 to 1.25:1. Conveniently, the possibility of applying these low molar ratios is advantageous, as it avoids the use of an excess of the compound of formula I and hence minimises the consumption of these usually expensive compounds. Suitably, the catalysts of the invention are prepared in a separate step preceding their use in-situ in the carbonylation reaction of a vinyl acetate compound.

Conveniently, the process of the invention may be carried out by dissolving the Group VIIIB metal or compound thereof as defined herein in a suitable solvent such as one of the hydroxyl group containing compounds or aprotic solvents previously described (a particularly preferred solvent would be the ester or acid product of the specific carbonylation reaction eg. Methyl lactate for vinyl acetate carbonylation) and subsequently admixing with a compound of formula I as defined herein.

The carbon monoxide may be used in the presence of other gases which are inert in the reaction. Examples of such gases include hydrogen, nitrogen, carbon dioxide and the noble gases such as argon.

Suitable Group VIIIB metals or a compound thereof which may be combined with a compound of formula I include cobalt, nickel, palladium, rhodium and platinum. Preferably, the Group VIIIB metal is palladium or a compound thereof. Suitable compounds of such Group VIII metals include salts of such metals with, or compounds comprising weakly coordinated anions derived from, nitric acid; sulphuric acid; lower alkanoic (up to C₁₂) acids such as acetic acid and propionic acid; sulphonic acids such as methane sulphonic acid, chlorosulphonic acid, fluorosulphonic acid, trifluoromethane sulphonic acid, benzene sulphonic acid, naphthalene sulphonic acid, toluene sulphonic acid, e.g. p-toluene sulphonic acid, t-butyl sulphonic acid, and 2-hydroxypropane sulphonic acid; sulphonated ion exchange resins; perhalic acid such as perchloric acid; ; halogenated carboxylic acids such as trichloroacetic acid and trifluoroacetic acid; orthophosphoric acid; phosphonic acids such as benzenephosphonic acid; and acids derived from interactions between Lewis acids and Broensted acids. Other sources which may provide suitable anions include the optionally halogenated tetraphenyl borate derivatives, e.g. perfluorotetraphenyl borate. Additionally, zero valent palladium complexes particularly those with labile ligands, e.g. triphenylphosphine or alkenes such as dibenzylideneacetone or styrene or tri(dibenzylideneacetone)dipalladium may be used.

The anion may be derived from or introduced as one or more of an acid having a pKa measured in aqueous solution at 18°C of less than 4, more preferably, less than 3, a salt with a cation that does not interfere with the reaction, e.g. metal salts or largely organic salts such as alkyl ammonium, and a precursor, such as an ester, that can break down under reaction conditions to generate the anion in situ. Suitable acids and salts include the acids and salts, other than unsubstituted carboxylates, listed supra.

The quantity of anion present is not critical to the catalytic behaviour of the catalyst system. The molar ratio of anion to palladium may be from 1:1 to 500:1, preferably from 2:1 to 100:1 and particularly from 3:1 to 30:1. Where the anion is provided by a combination of acid and salt, the relative proportion of the acid and salt is not critical. As mentioned, the catalyst system of the present invention may be used homogeneously or heterogeneously. Preferably, the catalyst system is used homogeneously.

The catalyst system of the present invention is preferably constituted in the liquid phase which may be formed by one or more of the reactants or by the use of a suitable solvent.

The molar ratio of the amount of vinyl acetate compound used in the reaction to the amount of hydroxyl providing compound is not critical and may vary between wide limits, eg. from 0.001:1 to 100:1 mol/mol.

The product of the reaction may be separated from the other components by any suitable means. However, it is an advantage of the present process that significantly fewer by-products are formed thereby reducing the need for further purification after the initial separation of the product as may be evidenced by the generally significantly higher selectivity. A further advantage is that the other components which contain the catalyst system which may be recycled and/or reused in further reactions with minimal supplementation of fresh catalyst.

Preferably, the carbonylation is carried out at a temperature of between -10 to 150°C, more preferably 0°C to 140°C, most preferably 20°C to 120°C. An especially preferred temperature is one chosen between 80°C to 120°C. Advantageously, the carbonylation can be carried out at moderate temperatures, it is particularly advantageous to be able to carry out the reaction at room temperature (20°C).

Preferably, when operating a low temperature carbonylation, the carbonylation is carried out between -30°C to 49°C, more preferably, -10°C to 45°C, still more preferably 0°C to 45°C, most preferably 10°C to 45°C. Especially preferred is a range of 10 to 35°C.

Preferably, the carbonylation is carried out at a CO partial pressure of between 0.80 x 10⁵ N.m⁻² -90 x 10⁵N.m⁻², more preferably 1 x 10⁵ N.m⁻²-65 x 10⁵N.m⁻², most preferably 1-30 x 10⁵ N.m⁻². Especially preferred is a CO partial pressure of 5 to 20 × 10⁵N.m-²,

Preferably, a low pressure carbonylation is also envisaged. Preferably, when operating a low pressure carbonylation the carbonylation is carried out at a CO partial pressure of between 0.1 to 5 x 10⁵N.m⁻² , more preferably 0.2 to 2 x10⁵N.m⁻² , most preferably 0.5 to 1.5 x10⁵N.m⁻².

As mentioned above, vinyl acetate can be substituted or non-substituted. However, it is preferred that the vinyl acetate is unsubstituted.

The use of stabilising compounds with the catalyst system may also be beneficial in improving recovery of metal which has been lost from the catalyst system. When the catalyst system is utilized in a liquid reaction medium such stabilizing compounds may assist recovery of the group VI or VlllB metal.

Preferably, therefore, the catalyst system includes in a liquid reaction medium a polymeric dispersant dissolved in a liquid carrier, said polymeric dispersant being capable of stabilising a colloidal suspension of particles of the group VI or VIIIB metal or metal compound of the catalyst system within the liquid carrier.

The liquid reaction medium may be a solvent for the reaction or may comprise one or more of the reactants or reaction products themselves. The reactants and reaction products in liquid form may be miscible with or dissolved in a solvent or liquid diluent.

The polymeric dispersant is soluble in the liquid reaction medium, but should not significantly increase the viscosity of the reaction medium in a way which would be detrimental to reaction kinetics or heat transfer. The solubility of the dispersant in the liquid medium under the reaction conditions of temperature and pressure should not be so great as to deter significantly the adsorption of the dispersant molecules onto the metal particles.

The polymeric dispersant is capable of stabilising a colloidal suspension of particles of said group VI or VIIIB metal or metal compound within the liquid reaction medium such that the metal particles formed as a result of catalyst degradation are held in suspension in the liquid reaction medium and are discharged from the reactor along with the liquid for reclamation and optionally for re-use in making further quantities of catalyst. The metal particles are normally of colloidal dimensions, e.g. in the range 5 - 100 nm average particle size although larger particles may form in some cases. Portions of the polymeric dispersant are adsorbed onto the surface of the metal particles whilst the remainder of the dispersant molecules remain at least partially solvated by the liquid reaction medium and in this way the dispersed group VI or VIIIB metal particles are stabilised against settling on the walls of the reactor or in reactor dead spaces and against forming agglomerates of metal particles which may grow by collision of particles and eventually coagulate. Some agglomeration of particles may occur even in the presence of a suitable dispersant but when the dispersant type and concentration is optimised then such agglomeration should be at a relatively low level and the agglomerates may form only loosely so that they may be broken up and the particles redispersed by agitation.

The polymeric dispersant may include homopolymers or copolymers including polymers such as graft copolymers and star polymers.

Preferably, the polymeric dispersant has sufficiently acidic or basic functionality to substantially stabilise the colloidal suspension of said group VI or VIIIB metal or metal compound.

By substantially stabilise is meant that the precipitation of the group VI or VIIIB metal from the solution phase is substantially avoided.

Particularly preferred dispersants for this purpose include acidic or basic polymers including carboxylic acids, sulphonic acids, amines and amides such as polyacrylates or heterocycle, particularly nitrogen heterocycle, substituted polyvinyl polymers such as polyvinyl pyrrolidone or copolymers of the aforesaid.

Examples of such polymeric dispersants may be selected from polyvinylpyrrolidone, polyacrylamide, polyacrylonitrile, polyethylenimine, polyglycine, polyacrylic acid, polymethacrylic acid, poly(3-hydroxybutyricacid), poly-L-leucine, poly-L-methionine, poly-L-proline, poly-L-serine, poly-L-tyrosine, poly(vinylbenzenesulphonic acid) and poly(vinylsulphonic acid).

Preferably, the polymeric dispersant incorporates acidic or basic moieties either pendant or within the polymer backbone. Preferably, the acidic moieties have a dissociation constant (pKₐ) of less than 6.0, more preferably, less than 5.0, most preferably less than 4.5. Preferably, the basic moieties have a base dissociation constant (pK_{b}) being of less than 6.0, more preferably less than 5.0 and most preferably less than 4.5, pKₐ and pK_{b} being measured in dilute aqueous solution at 25°C.

Suitable polymeric dispersants, in addition to being soluble in the reaction medium at reaction conditions, contain at least one acidic or basic moiety, either within the polymer backbone or as a pendant group. We have found that polymers incorporating acid and amide moieties such as polyvinylpyrollidone (PVP) and polyacrylates such as polyacrylic acid (PAA) are particularly suitable. The molecular weight of the polymer which is suitable for use in the invention depends upon the nature of the reaction medium and the solubility of the polymer therein. We have found that normally the average molecular weight is less than 100,000. Preferably, the average molecular weight is in the range 1,000 - 200,000, more preferably, 5,000 - 100,000, most preferably, 10,000 - 40,000 e.g. Mw is preferably in the range 10,000 - 80,000, more preferably 20,000 - 60,000 when PVP is used and of the order of 1,000 - 10,000 in the case of PAA.

The effective concentration of the dispersant within the reaction medium should be determined for each reaction/catalyst system which is to be used.

The dispersed group VI or VIIIB metal may be recovered from the liquid stream removed from the reactor e.g. by filtration and then either disposed of or processed for re-use as a catalyst or other applications. In a continuous process the liquid stream may be circulated through an external heat-exchanger and in such cases it may be convenient to locate filters for the palladium particles in these circulation apparatus.

Preferably, the polymer:metal mass ratio in g/g is between 1:1 and 1000:1, more preferably, between 1:1 and 400:1, most preferably, between 1:1 and 200:1. Preferably, the polymer:metal mass ratio in g/g is up to 1000, more preferably, up to 400, most preferably, up to 200.

The following examples further illustrate the present invention.

### Preparation of 1,2-bis-(ditertbutylphosphinomethyl) benzene

The preparation of this ligand was carried out in the manner disclosed in WO 99/47528 in accordance with example 18.

### Preparation of 1,2-bis-(ditertbutylphosphinomethyl)ferrocene

Di-tertbutylphosphine (Aldrich, 0.616 ml, 3.33 mmol) was added to a solution of 1,2-bis(dimethylaminomethyl)ferrocene (Example 1, 0.5 g, 1.66 mmol) in anhydrous acetic acid (100 ml) under nitrogen and the resulting mixture is stirred at 80°C for 72 hours. The anhydrous acetic acid is removed *in vacuo* at approximately 70°C to yield the crude title product as an orange/yellow solid. The crude product is recrystallised from ethanol with cooling to -17°C, filtered and the filtrate washed with cold ethanol to yield the title compound as a pale yellow solid (0.365 g, 44%, 84°C).
¹H NMR (250 MHz; CDCl₃): δ4.4 (2H, d, J = 2Hz); 3.95(5H, s); 3.75 (1H, t, 2Hz); 2.8 (2H, dd, 12Hz, 2Hz); 2.6 (2H, dd, 12Hz, 2Hz); 1.1 (18H, m).
¹³C NMR (63 MHz; CDCl₃): δ86.73 (d, 5.46 Hz); 70.08 (d, 4.41 Hz); 69.4665(s); 63.75(s); 31.80 (d, 2Hz); 31.45 (d, 1.98Hz); 29.89 (d, 1.88 Hz).
³¹P NMR (101 MHz; CDCl₃): δ15.00 ppm.

| | |
|---|---|
| Elemental analysis: | Found: C:66.79%; H:9.57% |
| | Calculated: C:66.93%; H:9.63% |

### Preparation of 1,2 bis(diadamantylphosphinomethyl) benzene

The preparation of this ligand was carried out as follows.

### 1.1 Preparation of (1-Ad)₂P(O)Cl

Phosphorous trichloride (83 cm³, 0.98 mol) was added rapidly via cannula to a combination of aluminium chloride (25.0 g, 0.19 mol) and adamantane (27.2 g, 0.20 mol) affording a tan suspension. The reaction was heated to reflux. After 10 mins, a yellow-orange suspension was formed (colour due to P(O)Cl₃?). The reaction was refluxed for a total of 6 h. The excess PCl₃ was removed by distillation at atmospheric pressure (BP 75 °C). On cooling to ambient temperature, an orange solid was formed. Chloroform (250 cm³) was added yielding an orange suspension, which was cooled to 0 °C. Water (150 cm³) was added slowly: initially the suspension viscosity increased, but on full addition of water the viscosity lessened. From this point the reaction was no longer kept under an atmosphere of Ar. The suspension was Buchner filtered to remove the yellow-orange solid impurity. The filtrate consisted of a two phase system. The lower phase was separated using a separating funnel, dried over MgSO₄ and Buchner filtered. The volatiles were removed via rotary evaporation, drying finally *in-vacuo,* affording an off-white powder. Yield 35.0 g, 99 %. ³¹P NMR: □ = 85 ppm, 99 % pure. FW = 352.85.

### 1.2 Preparation of (1-Ad)₂PH

LiAIH₄ (2.54 g, 67.0 mmol) was added over 90 minutes to a chilled (-10 °C) solution of (1-Ad)₂P(O)Cl (10.00 g, 28.3 mmol) in THF. (120 cm³), The reaction was allowed to warm to ambient temperature then stirred for 20 h. The grey suspension was cooled to -10 °C. HCI (aq., 5 cm³ c. HCI in 50 cm³ degassed water) was added slowly via syringe (initially very slowly due to exotherm of reaction), yielding a two phase system, with some solid material in the lower phase. Further HCl (~ 5 cm³ HCl) was added to improve the separation of the layers. The upper phase was removed via flat ended cannula, dried over MgSO₄ and filtered via cannula. The volatiles were removed *in-vacuo* affording the product as a white powder, isolated in the glovebox. Yield 6.00 g, 70 %. ³¹P NMR: □ = 17 ppm, 100 % pure. FW = 302.44.

### 1.3 Preparation of (1-Ad)₂PCl

A solution of Ad₂PH-(10.5 g, 34.7 mmol) and DBU (6.12 cm³, 40.9 mmol) in toluene (250 cm³) was chilled to -10 °C. Phosgene solution (30.0 cm³, 56.7 mmol, was added slowly via cannula, transferring via a measuring cylinder. This afforded a highly viscous pale yellow suspension. Additional toluene (100 cm³) was added via cannula to lessen the viscosity and ease the stirring. The reaction was filtered via cannula affording a yellow filtrate. The residue was washed with additional toluene (2 × 100 cm³) and the washings combined with the original filtrate. The volatiles were removed *in-vacuo* affording a pale yellow solid, which was washed with pentane (2 × 30 cm³, washings practically colourless). The product was dried *in-vacuo* and isolated in the glovebox as a lemon yellow powder. Yield 7.84 g, 67 %. ³¹P NMR: □ = 139 ppm, 99+ % pure. FW = 336.88.

### 1.4 Preparation of 1,2-bis(di-1-adamantylphosphinomethyl)benzene

### 1.4.1 Preparation of DI-SODIO-ORTHO-XYLENE(DISOD)

Bu"Li (2.5 M in hexanes, 11.28 cm³, 28.2 mmol) was added dropwise via syringe over 15 minutes to a stirred suspension of NaOBu¹ (crushed, 2.71 g, 28.2 mmol), *o*-xylene (1.15 cm³, 9.4 mmol) and N,N,N',N' - tetramethyl ethylene diamine (TMEDA) (4.26 cm³, 28.2 mmol) in heptane (100 cm³). The reaction was heated at 60 °C for 2 h, then allowed to cool / settle, affording a bright orange solid (DISOD) and pale yellow solution. The solution was removed via cannula filtration and the solid washed with additional heptane (50 cm³) and dried *in-vacuo.* 90 % yield assumed, 8.47 mmol.

### 1.4.2 Reaction of DI-SODIO-ORTHO-XYLENE with 2 equiv (1-Ad)₂PCl

A suspension of DISOD (8.47 mmol) in Et₂O (100 cm³) was prepared at -78 °C. A suspension of Ad₂PCl (5.70 g, 16.9 mmol) in Et₂O (120 cm³) was stirred rapidly at -78 °C and added via wide-bore cannula to the DISOD suspension. The reaction was allowed to warm to ambient temperature and stirred for 18 h, affording a pale yellow turbid solution. Water (degassed, 100 cm ³) added via cannula affording a two phase system, with a great deal of white solid present (product) due to the low solubility of this material. The upper phase (Et₂O was removed via cannula. The solid in the aqueous phase was extracted using dichloromethane (200 cm³), forming two clear phases. The lower phase (CH₂Cl₂) was removed via cannula and combined with the original Et₂O phase. The volatiles were removed *in-vacuo* yielding a slightly sticky solid. The solid was washed with pentane (200 cm³) with attrition being performed, the washings being removed via cannula filtration. The white solid was dried *in-vacuo* and isolated in the glovebox as a friable white powder. Yield 3.5 g, 59 %. FW = 707.01.
³¹P {1H} NMR data:- δ 24 ppm.
¹H NMR data:- (400 MHz, CDCl₃, 298 K) δ 7.59-7.50 (m, 2H, Ar-*H*), 7.09-6,99 (m, 2H, Ar-*H*), 3.01 (d, 4H, ²J_{PH} = 3.2 Hz, C*H*₂), 2.07-1.57 (m, 60H, C₁₀*H*₁₅) ppm.
¹³C {¹H} NMR data:- (100 MHz, CDCl₃, 298 K) δ 139.4 (dd, J_{PC}= 10.7 Hz, J_{PC}= 2.3 Hz, ArC), 131.0 (d, J_{PC} = 16.8 Hz, Ar-*C*), 125.0 (s, Ar-*C*), 41.1 (d, ²J_{PC} = 10.7 Hz, Ad-*C*²), 37.2 (s, Ad-*C*⁴), 36.9 (d, ¹J_{PC} = 22.9 Hz, Ad-*C*¹), 28.8 (d, ³J_{PC} = 7.6 Hz, Ad-*C*³ 22.0 (dd, ¹J_{PC} = 22.9 Hz, ⁴J_{PC} = 3.1 Hz, CH₂).

### Vinyl Acetate Carbonylation Examples 1-6

### Example 1

Standard conditions were as follows : In an oxygen free (< 10 ppm O₂) environment Pd₂(dba)₃ (22.0mg, 0.04mmoles Pd) and 1,2-bis(di-*tert*-butylphosphinomethyl)benzene (95.0mg, 0.24mmoles) were weighed into a 500ml round bottom flask. To this under a protective atmosphere of nitrogen 300ml of degassed methanol was added, and the solution allowed to stir for one hour. Then methane sulphonic acid (16µl, 0.24mmoles,) was added, along with 0.34g poly vinyl pyrollidone (PVP) and 75ml vinyl acetate (VAM). This solution was added to an autoclave under vacuum, and heated to 85°C, when 10 bar of CO was added. The gas addition took the temperature to 100°C, where it was held, and the solution allowed to react for 3 hours. The solution was then cooled the pressure released, and the autoclave emptied. A GC sample was taken for analysis.

This experiment was repeated with the same ligand varying the amounts of reactants and the results are set out in Table 3, examples 1-5. Experiment 6 repeats the experiment and uses the adamantyl ligand. Experiment 7 involved the use of 1,2-bis (di-tert-butylphosphinomethyl) ferrocene as the ligand.

### Results

The ligands 1,2-bis(di-tert-butylphosphinomethyl)benzene and 1,2-bis(di-tert-butylphosphinomethyl)ferrocene when complexed to palladium (II) exhibited catalytic activity for the carbonylation of vinyl acetate. Both the linear and branched products were produced (methyl-2-acetoxy propanoate and methyl-3-acetoxy propanoate), which can be used as precursors to the industrially favourable methyl lactate and 1,3 propanediol. Both ligands produced an *iso* to normal ratio of two or three to one, with comparable rates. Instead of a high selectivity to the linear product based on the steric bulk of the ligands, the reaction favoured, the branched metal-alkyl intermediate which is the precursor to the hydrolysis/transesterification product methyl lactate.

Table 1 shows the effect of temperature on the yield and rate of the reaction for a carbonylation otherwise carried out in accordance with example 1. Specifically, Example 1 was repeated at varying temperatures and the results are shown in table 1.

**Table 1 The Effect of Temperature on conversion**

| Temperature °C | Conversion % | TON (moles product/ mole Pd/hr) |
|---|---|---|
| Room Temp (ca 23°C) | 5.7 | 1162 |
| 50 | 65 | 13260 |
| 80 | 73 | 14892 |
| 100 | 72 | 14688 |

Table 2 shows the effect of pressure on the rate and yield using in other respects the conditions and reactants of Example 1 .

| Table 2 The effect of pressure variation on the Catalysis | | | |
|---|---|---|---|
| Pressure (bar gauge, including vapour pressure of solvent | | Conversion % | TON (moles product/ mole Pd/hr) |
| 5 | | 50 | 10312 |
| 10 | | 76 | 15538 |
| 20 | | 83 | 17042 |

The experiments detailed in table 3 Examples 1-5 were carried out to analyse the effect of the exotherm and the level of various components on the outcome. The experiments were carried out as set out above but with the amounts of components shown in table 3. Generally, the experiments were carried out at 100°C and take account of the exotherm in maintaining this temperature. However, in examples 2 & 3 the exotherm was not taken into account which causes the experimental temperature to rise to 130 °C. Examples 6 and 7 followed the procedure of example 1 but with the reactant and solvent amounts shown and using the adamantyl and ferrocene ligand respectively.

**Table 3**

| Experiment No | Experimental Conditions | Conversion | % TON | Rate | i:n |
|---|---|---|---|---|---|
| | | (total) | | (moles i+n/mol Pd/hr) | |
| 1 | 22mg Pd, 95mg ligand, 300ml MeOH 16ul MeSO3H, 0.34g PVP, 75ml VAM 100°C, 3hr, 10bar CO (no exotherm) | 66 | 13464 | 4488 | 2:1 |
| 2 | 44mg Pd, 190 mg ligand, 300ml MeOH 32ul MeSO3H, 1.35g PVP, 50ml VAM 100°C, 3hr, 10bar CO (inc exotherm) | 55 | 3730 | 1243 | 3:1 |
| 3 | 44mg Pd, 190mg ligand, 300ml MeOH 64ul MeS03H, 1.35g PVP, 50ml VAM 100°C, 3hr, 10bar CO (inc exotherm) | 60 | 4070 | 1356 | 5:1 |
| 4 | 44mg Pd, 190mg ligand, 300ml MeOH 32ul MeS03H, 1.35g PVP, 50ml VAM 100°C, 3hr, 10bar CO (no exotherm) | 60 | 4070 | 1356 | 2:1 |
| 5 | 44mg Pd, 190mg ligand, 300ml MeOH 32ul MeSO3H, 0.0g PVP, 50ml VAM 100°C, 3hr, 10bar CO (no exotherm) | 83 | 5630 | 1876 | 2.5:1 |
| 6 | 22mg Pd, 85mg Ad ligand, 300ml MeOH 16ul MeSO3H, 0.34g PVP, 75ml VAM 100°C, 3hr, 10bar CO (no exotherm) | 46 | 9384 | 3128 | 2:1 |
| 7 | 22mg Pd, 121mg Me ligand, 300ml MeOH 32ul MeSO3H, 1.35g PVP, 50ml VAM | 62 | 8422 | 2807 | 2:1 |

| | | | | | |
|---|---|---|---|---|---|
| NB "ligand" is 1,2 -bis (di-tert-butylphosphinomethyl) benzene Me ligand is 1,2-bis (di-tert-butyl phosphinomethyl) ferrocene "Ad Ligand" is 1,2 bis (diadamantylphosphinomethyl) benzene Pd is Pd(dba), 19% Pd | | | | | |

Examples 8 and 9 were conducted at low temperature and pressure of CO but with a high catalyst concentration compared with the vinyl acetate. Surprisingly, high conversions were achieved under these conditions. Example 8 was carried out at 30°C and Example 9 at 40°C.

### Examples 8 and 9

### Low temperature, Low CO pressure, High catalyst concentration experiments

Standard conditions for low temperature, high catalyst concentration experiments were as follows : In an oxygen free (< 10 ppm O₂) environment Pd₂(dba)₃ 23.19% Pd (45.7mg, 0.1 mmoles Pd) and 1,2-bis(di-*tert*-butylphosphinomethyl)benzene (157.7mg, 0.4mmoles) were weighed into a 250ml round bottom flask. To this under a protective atmosphere of nitrogen was added methanol (23ml), vinyl acetate (2ml), toluene (530µl) and methanesulphonic acid (29µl, 0.4mmoles). This solution was warmed until all of the solid had dissolved and the flask was then equipped with a cardice condenser and placed in a water bath. The reaction was stirred by means of a magnetic follower and allowed to equilibrate at the water bath set point.

Carbon monoxide was bubbled into the stirred solution for a period of 1 minute, by means of a needle introduced via a suba seal in the reaction flask. After this, without altering the carbon monoxide flow rate, the needle was withdrawn from the liquid and the needle was kept well above the liquid level for the remainder of the experiment. In example 8 conducted at 30°C samples were taken at 30 minute intervals for 2 hours and then at hourly intervals up to 5 hours. In example 9 conducted at 40°C samples were taken after 0.5, 1 and 3 hours. The samples were stored at -20C prior to analysis by GC. The results are illustrated in tables 1.0 and 2.0 below.

### Example 8 30°C

| Time (hours) | Conversion | Ratio I:b |
|---|---|---|
| 0 | 0 | 0 |
| 0.5 | 14.5 | 3.2:1 |
| 1.0 | 24.5 | 3.2:1 |
| 1.5 | 39.3 | 3.4:1 |
| 2 | 46.0 | 3.4:1 |
| 3 | 65.3 | 3.4:1 |
| 4 | 73.1 | 3.4:1 |
| 5 | 81.4 | 3.4:1 |

### Example 9 40°C

| Time (hours) | Conversion | Ratio I:b |
|---|---|---|
| 0 | 0 | 0 |
| 0.5 | 33.5 | 3.3:1 |
| 1.0 | 39.6 | 3.2:1 |
| 3.0 | 70.4 | 3.2:1 |

After distillation of the products of the carbonylation, 2-acetoxy methyl propionate and 3-acetoxy methyl propionate were collected as different distillates.

### Production of Lactate and 3-hydroxy Esters

### Preparation of 3 hydroxymethylpropionate

To 25g of 3 acetoxy methyl propionate (0.171 moles) was added 25g MeOH (0.78 moles) containing 1%w/w methane sulphonic acid. The solution was stirred at 60C for six hours before cooling to room temperature. The sample was analysed by GC, the peak corresponding to 3 acetoxy methylpropionate had completely disappeared and been replaced by a peak corresponding to 3 hydroxymethylpropionate.

### Preparation of 2-hydroxymethylpropionate

To 25g of 2 acetoxy methylpropionate (0.171 moles) was added 25g MeOH (0.78 moles) containing 1 %w/w methane sulphonic acid. The solution was stirred at 60C for six hours before cooling to room temperature. The sample was analysed by GC, the peak corresponding to 2 acetoxy methylpropionate had completely disappeared and been replaced by a peak corresponding to 2 hydroxymethylpropionate.

### Preparation of 2 hydroxy propionic acid (lactic acid)

To 25g of 2 acetoxy methyl propionate (0.171 moles) was added 25g MeOH. To this stirred solution was added 20g sodium hydroxide (0.5 moles) dissolved in 20 ml of water. The solution was stirred for one hour at 50C before cooling to room temperature. The pH of the solution was then adjusted to pH 3.0 by the slow addition of HCl and the sample stirred for 1 hour. The sample was analysed by GC, the peak corresponding to 2 acetoxy methyl propionate had completely disappeared and been replaced by a peak corresponding to 2 hydroxy propionic acid.

### Preparation of 3 hydroxy propionic acid

To 25g of 3 acetoxy methyl propionate (0.171 moles) was added 25g MeOH. To this stirred solution was added 20g sodium hydroxide (0.5 moles) dissolved in 20 ml of water. The solution was stirred for one hour at 50C before cooling to room temperature. The pH of the solution was then adjusted to pH 3.0 by the slow addition of HCl and the sample stirred for 1 hour. The sample was analysed by GC, the peak corresponding to 3 acetoxy methyl propionate had completely disappeared and been replaced by a peak corresponding to 3 hydroxy propionic acid.

## Claims

1. A process for the production of a lactate ester or acid of formula II 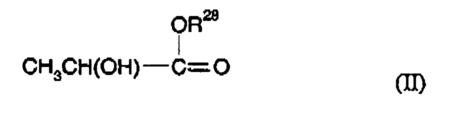 comprising the steps of carbonylating a vinyl acetate compound of formula (IV)
R²⁹ - C(O) O CR³⁰ = CR³¹ R³²
wherein R²⁹ may be selected from hydrogen, C₁ - C₁₀ alkyl, aryl, Het, halo, cyano, nitro, OR¹⁹, OC(O)R²⁰, C(O)R²¹, C(O)OR²², NR²³ R²⁴, C(O)NR²⁵R²⁶, C(S) R²⁵R²⁶SR²⁷, C(O) SR²⁹ wherein R¹² -R¹⁸ and R¹⁹-R²⁷ are as defined below and R³⁰R³² represent hydrogen,
with carbon monoxide in the presence of a source of hydroxyl groups and of a catalyst system, the catalyst system obtainable by combining:
(a) a metal of Group VIII B or a compound thereof; and
(b) a bidentate phosphine of general formula (I) 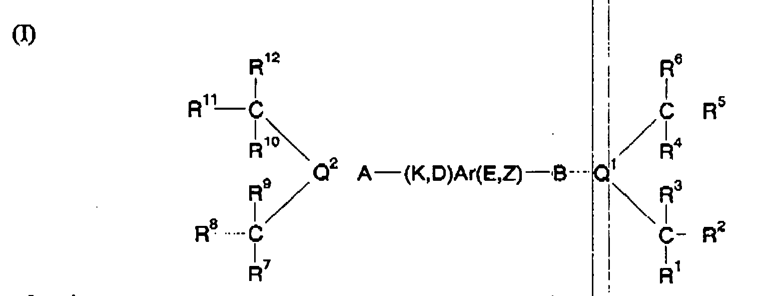
wherein:
Ar is a bridging group comprising an optionally substituted aryl moiety to which the phosphorus atoms are linked on available adjacent carbon atoms;
A and B each independently represent C₁-C₁₀ alkylene;
K, D, E and Z are substituents of the aryl moiety (Ar) and each independently represent hydrogen, C₁-C₁₀ alkyl, aryl, Het, halo, cyano, nitro, OR¹⁹, OC(O)R²⁰, C(O)R²¹, C(O)OR²², NR²³R²⁴, C(O)NR²⁵R²⁶, C(S)R²⁵R²⁶, SR²⁷, C(O)SR²⁷, or-J-Q³(CR¹³(R¹⁴)(R¹⁵))CR¹⁶(R¹⁷)(R¹⁸) where J represents C₁-C₁₀ alkylene; or two adjacent groups selected from K, Z, D and E together with the carbon atoms of the aryl ring to which they attached form a further phenyl ring, which is optionally substituted by one or more substituents selected from hydrogen, C₁-C₁₀ alkyl, halo, cyano, nitro, OR¹⁹, OC(O)R²⁰, C(O)R²¹, C(O)OR²², NR²³R²⁴, C(O)NR²⁵R²⁶, C(S)R²⁵R²⁶, SR²⁷ or C(O)SR²⁷ or, when Ar is a cyclopentadienyl group, Z may be represented by -M(L₁)ₙ (L₂)ₘ and Z is connected via a metal ligand bond to the cyclopentadienyl group;
R¹ to R¹⁸ each independently represent C₁-C₁₀ alkyl, aryl, or Het;
R¹⁹ to R²⁷ each independently represent hydrogen, C₁-C₁₀ alkyl, aryl or Het;
M represents a Group VIB or VIITB metal or metal cation thereof;
L₁ represents a cyclopentadienyl, indenyl or aryl group each of which groups are optionally substituted by one or more substituents selected from hydrogen, C₁-C₁₀ alkyl, halo, cyano, nitro, OR¹⁹, OC(O)R²⁰, C(O)R²¹, C(O)OR²², NR²³R²⁴, C(O)NR²⁵R²⁶, C(S)R²⁵R²⁶, SR²⁷, C(O)SR²⁷ or ferrocenyl;
L₂ represents one or more ligands each of which are independently selected from hydrogen, C₁-C₁₀ alkyl, alkylaryl, halo, CO, PR⁴³R⁴⁴R⁴⁵ or NR⁴⁶R⁴⁷R⁴⁸; C₁-C₁₀ alkyl, alkylaryl, halo, CO, PR⁴³R⁴⁴R⁴⁵ or N⁴⁶R⁴⁷R⁴⁸;
R⁴³ to R⁴⁸ each independently represent hydrogen, C₁-C₁₀ alkyl, aryl or Het;
**n= 0 or 1;**
and m = 0 to 5;
provided that when n = 1 then m equals 0, and when n equals 0 then m does not equal 0;
Q¹, Q² and Q³ (when present) each independently represent phosphorous, arsenic or antimony and in the latter two cases references to phosphine or phosphorus above are amended accordingly,
to produce a product comprising a branched (iso) product and chemically treating the said branched (iso) product to produce the corresponding lactate or acid of formula II, wherein R²⁵ is selected from H, or a C₁-C₃₀ alkyl or aryl moiety which may be substituted or unsubstituted and either branched or linear.

2. A process according to any preceding claim, wherein the ratio of branched :linear product from the carbonylation process is greater than 1.5:1.

3. A process according to any preceding claim, wherein the Group VIII B metal is palladium.

4. A process according to any preceding claim, wherein the linear (n) and branched (iso) products of the carbonylation may be separated either before or after the treatment step.

5. A process according to any preceding claim, wherein the products of the reaction are separated by distillation.

6. A process according to any preceding claim, wherein when K, D, E or Z represent -J-Q³(CR¹³(R¹⁴)(R¹⁵))CR¹⁶(R¹⁷)(R¹⁹), the respective K, D, E or Z is on the aryl carbon adjacent the aryl carbon to which A or B is connected or, if not so adjacent, is adjacent a remaining K, D, E or Z group which itself represents -J-Q³(CR¹³(R¹⁴)(R¹⁵))CR¹⁶(R¹⁷)(R¹⁸)·

7. A process according to any preceding claim, wherein the carbon monoxide may be used in pure form or diluted with an inert gas such as nitrogen, carbon dioxide or a noble gas such as argon.

8. A process according to any preceding claim, wherein the ratio (volume/volume) of vinyl acetate compound to hydroxyl group containing compound lies in the range of 1:0. to 1:10.

9. A process according to any preceding claim, wherein the amount of Group VIII B metal is in the range 10⁻⁷ to 10⁻¹ moles per mole of vinyl acetate compound.

10. A process according to any preceding claim, wherein the carbonylation of a vinyl acetate compound is performed in one or more aprotic solvents.

11. A process according to claim 10, wherein the aprotic solvent has a dielectric constant that is below 50 at 298.15 K and at 1x10⁵ Nm⁻².

12. A process according to any preceding claim, wherein the catalyst compounds act as a heterogeneous catalyst.

13. A process according to any of claims 1 to 11, wherein the catalyst compounds act as a homogeneous catalyst.

14. A process according to claim 12 wherein the process is carried out with the catalyst comprising a support.

15. A process according to claim 14, wherein the support is insoluble.

16. A process according to either claim 14 or claim 15, wherein the support comprises a polymer such as a polyolefin, polystyrene or polystyrene copolymer such as a divinylbenzene copolymer; a silicon derivative such as a functionalised silica, a silicone or a silicone rubber, or porous inorganic oxides and inorganic chlorides.

17. A process according to any preceding claim, wherein the carbonylation is carried out at a temperature of between -10 to 150°C.

18. A process according to any preceding claim, wherein the carbonylation is carried out at a CO partial pressure of between 0.80 x 10⁵ N.m⁻²-90 x 10⁵N.m⁻².

19. A process according to any of claims 1 to 17, wherein the carbonylation is carried out at a low CO partial pressure of between 0.1 to 5 x 10⁵N.m⁻².

20. A process according to any preceding claim, wherein the bidentate phosphine is independently selected from any of the following: bis (di-t-butyl phosphino)-o-xylene (also known as 1,2 bis (di-t-butylphosphinomethyl) benzene); 1,2 bis (diadamantylphosphinomethyl) benzene; 1,2 bis (diadamantylphosphinomethyl) naphthalene; 1,2 bis (di-t-pentyl phosphino)-o-xylenc (also known as 1,2 [bis (di-t-pentyl-phosphinomethyl) benzene); bis 2,3 (di-t-butyl phosphinomethyl) naphthalene; 1,2-bis-(ditertbutylphosphinomethyl) ferrocene;1,2,3-tris-ditertbutylphosphinomethyl) ferrocene;1,2 bis (diadamantylphosphinomethyl) ferrocene; and 1,2 bis (di-t-pentyl phosphinomethyl) ferrocene.

21. The use of the catalyst system according to any preceding claim for the production of a lactate ester or acid of formula (II) the said production comprising the steps of carbonylation of a vinyl acetate compound of formula (IV) followed by treatment of the branched (iso) product of the carbonylation to produce the ester or acid.

22. The use of a catalyst system as defined in any of claims 1 to 20, wherein the catalyst is attached to a support.

23. The use of a catalyst according to claim 21, wherein the treatment is hydrolysis or transesterification.

24. The use of the catalyst according to claim. 23, wherein the product is hydrogenated subsequent to hydrolysis.

## Patentansprüche

1. Verfahren zur Herstellung eines Lactatesters bzw. einer Säure der Formel II 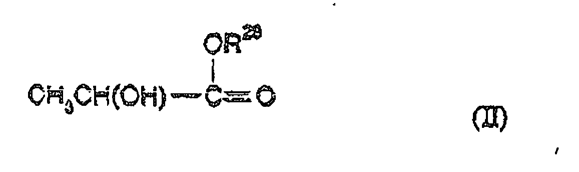 umfassend die Schritte der Carbonylierung einer Vinylacetatverbindung der Formel (IV)
**R²⁹-C(O)O CR¹⁰ CR³¹ R³²**,
in welcher R²⁹ ausgewählt sein kann aus Wasserstoff, C₁-C₁₀-Alkyl, Aryl, Het, Halogen, Cyano, Nitro, OR¹⁹, OC(O)R²⁰, C(O)R²¹, C (O) OR²², NR²³R²⁴, C (O) NR²⁵R²⁶, C(S)R²⁵R²⁶, SR²⁷, C(O)SR²⁹, wobei R¹²-R¹⁸ und R¹⁹-R²⁷ wie unten definiert sind und R³⁰-R³² für Wasserstoff stehen,
mit Kohlenmonoxid in Gegenwart einer Hydroxylgruppenquelle und eines Katalysatorsystems, wobei das Katalysatorsystem erhältlich ist durch Kombinieren:
(a) eines Metalls der Gruppe VIII B oder einer Verbindung davon und
(b) eines zweizähnigen Phosphins der allgemeinen Formel (I) 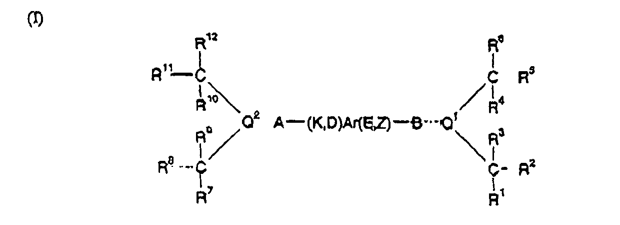
wobei:
Ar für eine verbrückende Gruppe steht, die eine gegebenenfalls substituierte Aryleinheit umfaßt, an die die Phosphoratome an verfügbare benachbarte Kohlenstoffatome gebunden sind;
A und B jeweils unabhängig voneinander für C₁-C₁₀-Alkylen stehen;
K, D, E und Z Substituenten der Aryleinheit (Ar) sind und jeweils unabhängig voneinander für Wasserstoff, C₁-C₁₀-Alkyl, Aryl, Het, Halogen, Cyano, Nitro, OR¹⁹, OC(O)R²⁰, C(O) R²¹, C(O)OR²², NR²³R²⁴, C (O) NR²⁵R²¹ C(S)R²⁵R²⁶, SR²⁷, C(O) SR²⁷ oder J-Q³-(CR¹³ (R¹⁴) (R¹⁵)) CR¹⁶ (R¹⁷) (R¹⁸) stehen, wobei J für C₁-C₁₀-Alkylen steht; oder zwei benachbarte Gruppen ausgewählt aus K, Z, D und E zusammen mit den Kohlenstoffatomen des Arylrings, an die sie gebunden sind, einen weiteren Phenylring bilden, der gegebenenfalls substituiert ist durch einen oder mehrere Substituenten ausgewählt aus Wasserstoff, C₁-C₁₀-Alkyl, Halogen, Cyano, Nitro, OR¹⁹, OC (O) R²⁰, C (O) R²¹, C(O)OR²², NR²³R²⁴, C(O)NR²⁵R²⁶, C(S)R²⁵R²⁶, SR²⁷ oder C(O)SR²⁷ oder, wenn Ar für eine Cyclopentadienylgruppe steht, Z durch -M (L₁)ₙ (L₂) m wiedergegeben werden kann und Z über eine Metall-Ligand-Bindung mit der Cyclopentaldienylgruppe verbunden ist;
R¹ bis R¹⁸ jeweils unabhängig voneinander für C₁-C₁₀-Alkyl, Aryl, oder Het stehen;
R¹⁹ bis R²⁷ jeweils unabhängig voneinander für Wasserstoff, C₁-C₁₀ Alkyl, Aryl, oder Het stehen;
M für ein Metall der Gruppe VIB oder VIII B oder ein Metallkation davon steht;
L₁ für eine Cyclopentadienyl-, Indenyl- oder Arylgruppe steht, wobei diese Gruppen jeweils gegebenenfalls durch einen oder mehrere Substituenten ausgewählt aus Wasserstoff, C₁-C₁₀-Alkyl, Halogen, Cyano, Nitro, OR¹⁹, OC (O) R²⁰, C (O) R²¹, C(O)OR ²² NR²³ R²⁴ C (O) NR²⁵R²⁶, C (S) R²⁵R²⁶, SR²⁷ C(O)SR²⁷ oder Ferrocenyl substituiert sind;
L₂ für einen oder mehrere Liganden steht, die jeweils unabhängig voneinander ausgewählt sind aus Wasserstoff, C₁-C₁₀-Alkyl, Alkylaryl, Halogen, CO_{,} PR⁴³R⁴⁴R⁴⁵ oder NR⁴⁶R⁴⁷R⁴⁸;
R⁴³ bis R⁴⁸ jeweils unabhängig voneinander für Wasserstoff, C₁-C₁₀-Alkyl, Aryl oder Het stehen;
n = 0 oder 1; sind
und m = 0 bis 5;
mit der Maßgabe, daß, wenn n = 1 ist, m = 0 ist, und daß, wenn n gleich 0 ist, m nicht gleich 0 ist;
Q¹, Q² und Q³ (falls vorhanden) jeweils unabhängig voneinander für Phosphor, Arsen oder Antimon stehen und in den beiden letztgenannten Fällen obige Verweise auf Phosphin bzw. Phosphor entsprechend zu ergänzen sind,
unter Herstellung eines Produkts, das ein verzweigtes (Iso)produkt umfaßt, und der chemischen Behandlung dieses verzweigten (Iso)produkts unter Herstellung des entsprechenden Lactats bzw. der entsprechenden Säure der Formel II, wobei R²⁵ ausgewählt ist aus H oder einer C₁-C₃₀-Alkyl- oder Aryleinheit, die substituiert oder unsubstituiert und entweder verzweigt oder geradkettig sein kann.

2. Verfahren nach Anspruch 1, wobei das Verhältnis von verzweigtem zu geradkettigem Produkt aus dem Carbonylierungsverfahren größer als 1,5:1 ist.

3. Verfahren nach Anspruch 1 oder 2, wobei es sich bei dem Metall der Gruppe VIII B um Palladium handelt.

4. Verfahren nach einem der vorhergehenden Ansprüche, wobei die geradkettigen (n-) und verzweigten (Iso-) Produkte der Carbonylierung entweder vor oder nach dem Behandlungsschritt getrennt werden können.

5. Verfahren nach einem der vorgehenden Ansprüche, wobei die Produkte der Umsetzung durch Destillation getrennt werden.

6. Verfahren nach einem der vorgehenden Ansprüche, wobei wenn K, D, E oder Z für -J-Q³-(CR¹³ (R¹⁴) (R¹⁵)) CR¹⁶ (R¹⁷) (R¹⁸) steht, sich der entsprechende K-, D-, E- bzw. Z-Rest an dem Arylkohlenstoff befindet, der dem Arylkohlenstoff benachbart ist, an den A oder B gebunden ist, oder, wenn er nicht so benachbart ist, zu einer verbliebenen K-, D-, E- oder Z-Gruppe benachbart ist, die selbst für -J-Q³- (CR¹³ (R¹⁴) (R¹⁵)) CR¹⁶ (R¹⁷) (R¹⁸) steht.

7. Verfahren nach einem der vorhergehenden Ansprüche, wobei das Kohlenmonoxid in reiner Form oder verdünnt mit einem inerten Gas wie Stickstoff, Kohlenstoffdioxid oder einem Edelgas wie Argon verwendet werden kann.

8. Verfahren nach einem der vorhergehenden Ansprüche, wobei das Volumenverhältis von Vinylacetatverbindung zu die Hydroxylgruppe enthaltender Verbindung im Bereich von 1:0,1 zu 1:10 liegt.

9. Verfahren nach einem der vorhergehenden Ansprüche, wobei die Menge an Metall der Gruppe VIII B im Bereich von 10⁻⁷ bis 10⁻¹ mol pro Mol an Vinylacetatverbindung liegt.

10. Verfahren nach einem der vorhergehenden Ansprüche, wobei die Carbonylierung einer Vinylacetatverbindung in einem oder mehreren aprotischen Lösungsmitteln durchgeführt wird.

11. Verfahren nach Anspruch 10 , wobei das aprotische Lösungsmittel eine Dielektrizitätskonstante aufweist, die bei 298,15 K und bei 1 x 10⁵ Nm⁻² unter 50 liegt.

12. Verfahren nach einem der vorhergehenden Ansprüche, wobei die Katalysatorverbindungen als heterogener Katalysator wirken.

13. Verfahren nach einem der Ansprüche 1 bis 11, wobei die Katalysatorverbindungen als homogener Katalysator wirken.

14. Verfahren nach Anspruch 12, wobei das Verfahren mit einem geträgerten Katalysator durchgeführt wird.

15. Verfahren nach Anspruch 14, wobei der Träger unlöslich ist.

16. Verfahren nach Anspruch 14 oder 15, wobei der Träger ein Polymer wie z.B. ein Polyolefin, ein Polystyrol oder Polystyrolcopolymer, wie etwa ein Divinylbenzolcopolymer; ein Siliciumderivat wie etwa ein funktionalisierte Kieselsäure, ein Silikon oder Silikongummi; oder poröse anorganische Oxide und anorganische Chloride umfaßt.

17. Verfahren nach einem der vorhergehenden Ansprüche, wobei die Carbonylierung bei einer Temperatur von -10 bis 150°C durchgeführt wird.

18. Verfahren nach einem der vorhergehenden Ansprüche, wobei die Carbonylierung bei einem CO-Teildruck von zwischen 0, 80 x 10⁵ Nm⁻² -90 x 10⁵ Nm⁻² durchgeführt wird.

19. Verfahren nach einem der Ansprüche 1 bis 17, wobei die Carbonylierung bei einem niedrigen CO-Teildruck von 0,1 bis 5 x 10⁵ Nm⁻² durchgeführt wird.

20. Verfahren nach einem der vorhergehenden Ansprüche, wobei das zweizähnige Phosphin unabhängig aus den folgenden ausgewählt ist: Bis-(di-t-butylphosphino)-o-xylol (auch bekannt als 1,2-Bis-(di-t-butylphosphinomethyl)benzol); 1,2-Bis-(diadamantylphosphinomethyl)benzol; 1,2-Bis-(diadamantylphosphinomethyl)naphthalin, 1,2-Bis-(di-t-pentylphosphino)-o-xylol (auch bekannt als 1,2-Bis-(di-t-pentylphosphinomethyl)benzol); Bis-2,3-(di-t-butylphosphinomethyl)naphthalin; 1,2-Bis-(di-tert.-butylphosphinomethyl)ferrocen; 1,2,3-Tris-(di-tert.-butylphosphinomethyl)ferrocen; 1,2-Bis-(diadamantylphosphinomethyl)ferrocen; und 1,2-Bis-(di-t-pentylphosphinomethyl)ferrocen.

21. Verwendung des Katalysatorsystems nach einem der vorhergehenden Ansprüche zur Herstellung eines Lactatesters bzw. einer Säure der Formel (II), wobei die Herstellung die Schritte der Carbonylierung einer Vinylacetatverbindung der Formel (IV), gefolgt von der Behandlung des verzweigten (Iso)produkts der Carbonylierung zur Herstellung des Esters bzw. der Säure umfaßt.

22. Verwendung eines Katalysatorsystems nach einem der Ansprüche 1 bis 20, wobei der Katalysator geträgert ist.

23. Verwendung eines Katalysators nach Anspruch 21, wobei es sich bei der Behandlung um eine Hydrolyse oder eine Umesterung handelt.

24. Verwendung des Katalysators nach Anspruch 23, wobei das Produkt im Anschluß an die Hydrolyse hydriert wird.

## Revendications

1. Procédé de production d'un ester lactate ou de l'acide lactique de formule II 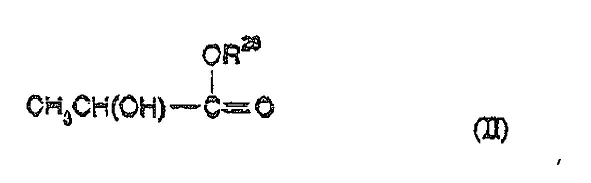 comprenant les étapes de carbonylation d'un composé acétate de vinyle de formule (IV)
R²⁹-C(O) O CR¹⁰=CR³¹ R³²,
dans laquelle R²⁹ peut être choisi parmi un atome d'hydrogène, un groupe alkyle en C₁-C₁₀, un groupe aryle, un groupe Het, un groupe halogéno, un groupe cyano, un groupe nitro, un groupe OR¹⁹, un groupe OC (O) R²⁰, un groupe C (O) R²¹, un groupe C (O) OR²², un groupe NR²³R²⁴, un groupe C (O) NR²⁵R²⁶, un groupe C (S) R²⁵R²⁶, un groupe SR²⁷, un groupe C (O) SR²⁹, dans lesquels R¹² à R¹⁸ et R¹⁹ à R²⁷ sont tels que définis ci-dessous et R³⁰ à R³² représentent un atome d'hydrogène,
avec du monoxyde de carbone en présence d'une source de groupes hydroxyle et d'un système catalytique, le système catalytique pouvant être obtenu en combinant :
(a) un métal du groupe VIII B ou un composé de celui-ci ; et
(b) une phosphine bidentée de formule générale (I) 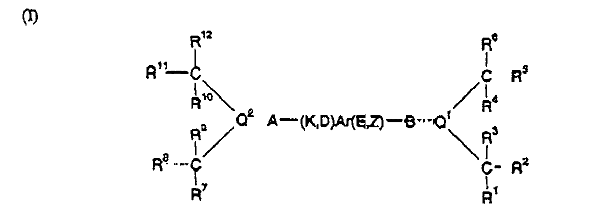
dans laquelle :
Ar est un groupe de liaison renfermant un fragment aryle éventuellement substitué auquel les atomes de phosphore sont liés sur des atomes de carbone adjacents disponibles ;
A et B représentent chacun indépendamment un groupe alkylène en C₁-C₁₀;
K, D, E et Z sont des substituants du fragment aryle (Ar) et représentent chacun indépendamment un atome d'hydrogène, un groupe alkyle en C₁-C₁₀, un groupe aryle, un groupe Het, un groupe halogéno, un groupe cyano, un groupe nitro, un groupe OR¹⁹, un groupe OC(O)R²⁰, un groupe C(O)R²¹, un groupe C(O)OR²², un groupe NR²³R²⁴, un groupe C(O) NR²⁵R²⁶, un groupe C(S)R²⁵R²⁶ un groupe SR²⁷, un groupe C(O)SR²⁷ ou un groupe J-Q³-(CR¹³(R¹⁴) (R¹⁵)) CR¹⁶(R¹⁷) (R¹⁸), dans lequel J représente un groupe alkylène en C₁-C₁₀; ou deux groupes adjacents choisis parmi K, Z, D et E, conjointement avec les atomes de carbone du noyau aryle auxquels ils sont liés, forment un noyau phényle supplémentaire qui est éventuellement substitué par un ou plusieurs substituants choisis parmi un atome d'hydrogène, un groupe alkyle en C₁-C₁₀, un groupe halogéno, un groupe cyano, un groupe nitro, un groupe OR¹⁹, un groupe OC(O)R²⁰, un groupe C(O)R²¹, un groupe C (O) OR²², un groupe NR²³R²⁴, un groupe C(O) NR²⁵R²⁶, un groupe C(S)R²⁵R²⁶, un groupe SR²⁷ ou un groupe C(O)SR²⁷ ou, lorsque Ar est un groupe cyclopentadiényle, Z peut être représenté par un groupe -M(L₁)ₙ(L₂)ₘ et Z est relié par l'intermédiaire d'une liaison de ligand métallique au groupe cyclopentadiényle ;
R¹ à R¹⁸ représentent chacun indépendamment un groupe alkyle en C₁-C₁₀, un groupe aryle ou un groupe Het ;
R¹⁹ à R²⁷ représentent chacun indépendamment un atome d'hydrogène, un groupe alkyle en C₁-C₁₀, un groupe aryle ou un groupe Het ;
M représente un métal du groupe VIB ou VIIIB ou un cation métallique de celui-ci ;
L₁ représente un groupe cyclopentadiényle, un groupe indényle ou un groupe aryle, chacun de ces groupes étant éventuellement substitué par un ou plusieurs substituants choisis parmi un atome d'hydrogène, un groupe alkyle en C₁-C₁₀, un groupe halogéno, un groupe cyano, un groupe nitro, un groupe OR¹⁹, un groupe OC(O)R²⁰ un groupe C(O)R²¹, un groupe C(O)OR²², un groupe NR²³R²⁴, un groupe C(O) NR²⁵R²⁶, un groupe C (S) R²⁵R²⁶ un groupe SR²⁷ un groupe C(O)SR²⁷ ou un groupe ferrocényle ;
L₂ représente un ou plusieurs ligands, dont chacun est choisi indépendamment parmi un atome d'hydrogène, un groupe alkyle en C₁-C₁₀, un groupe alkylaryle, un groupe halogéno, un groupe CO, un groupe PR⁴³R⁴⁴R⁴⁵ ou un groupe NR⁴⁶R⁴⁷R⁴⁸ ;
R⁴³ à R⁴⁸ représentent chacun indépendamment un atome d'hydrogène, un groupe alkyle en C₁-C₁₀, un groupe aryle ou un groupe Het ;
n vaut 0 ou 1 ;
et m vaut de 0 à 5 ;
à condition que lorsque n vaut 1, alors m vaille 0, et que lorsque n vaut 0, alors m ne vaille pas 0 ;
Q¹, Q² et Q³ (lorsqu'ils sont présents) représentent chacun indépendamment un atome de phosphore, un atome d'arsenic ou un atome d'antimoine, et dans ces deux derniers cas, les références à une phosphine ou à un atome de phosphore ci-dessus sont modifiées en conséquence,
en vue de produire un produit comprenant un produit ramifié (iso), et de traitement chimique dudit produit ramifié (iso) en vue de produire le lactate ou l'acide correspondant de formule II, dans laquelle R²⁸ est choisi parmi un atome d'hydrogène, un fragment alkyle en C₁-C₃₀ ou un fragment aryle qui peut être substitué ou non substitué et soit ramifié, soit linéaire.

2. Procédé selon l'une quelconque des revendications précédentes, dans lequel le rapport entre le produit ramifié et le produit linéaire du procédé de carbonylation est supérieur à 1,5:1.

3. Procédé selon l'une quelconque des revendications précédentes, dans lequel le métal du groupe VIIIB est le palladium.

4. Procédé selon l'une quelconque des revendications précédentes, dans lequel les produits linéaire (n) et ramifié (iso) de la carbonylation peuvent être séparés soit avant, soit après l'étape de traitement.

5. Procédé selon l'une quelconque des revendications précédentes, dans lequel les produits de la réaction sont séparés par distillation.

6. Procédé selon l'une quelconque des revendications précédentes, dans lequel lorsque K, D, E ou Z représentent un groupe -J-Q³ (CR¹³ (R¹⁴) (R¹⁵)) CR¹⁶ (R¹⁷) (R¹⁸) le groupe K, D, E ou Z respectif est sur l'atome de carbone de groupe aryle adjacent à l'atome de carbone de groupe aryle auquel A ou B est relié ou, s'il n'est pas ainsi adjacent, est adjacent à un groupe K, D, E ou Z restant qui représente lui-même un groupe -J-Q³ (CR¹³ (R¹⁴) (R¹⁵)) CR¹⁶ (R¹⁷) (R¹⁸).

7. Procédé selon l'une quelconque des revendications précédentes, dans lequel le monoxyde de carbone peut être utilisé sous une forme pure ou dilué avec un gaz inerte tel que l'azote, le dioxyde de carbone ou un gaz noble tel que l'argon.

8. Procédé selon l'une quelconque des revendications précédentes, dans lequel le rapport (volume/volume) entre le composé acétate de vinyle et le composé renfermant un groupe hydroxyle est dans la plage de 1:0,1 à 1:10.

9. Procédé selon l'une quelconque des revendications précédentes, dans lequel la quantité de métal du groupe VIIIB est dans la plage de 10⁻⁷ à 10⁻¹ mol par mole de composé acétate de vinyle.

10. Procédé selon l'une quelconque des revendications précédentes, dans lequel la carbonylation d'un composé acétate de vinyle est réalisée dans un ou plusieurs solvants aprotiques.

11. Procédé selon la revendication 10, dans lequel le solvant aprotique présente une constante diélectrique qui est inférieure à 50 à 298,15 K et à 1 x 10¹ Nm⁻².

12. Procédé selon l'une quelconque des revendications précédentes, dans lequel les composés catalytiques agissent en tant que catalyseur hétérogène.

13. Procédé selon l'une quelconque des revendications 1 à 11, dans lequel les composés catalytiques agissent en tant que catalyseur homogène.

14. Procédé selon la revendication 12, le procédé étant réalisé avec le catalyseur comprenant un support.

15. Procédé selon la revendication 14, dans lequel le support est insoluble.

16. Procédé selon la revendication 14 ou la revendication 15, dans lequel le support comprend un polymère tel qu'une polyoléfine, un polystyrène ou un copolymère de polystyrène tel qu'un copolymère de divinylbenzène ; un dérivé du silicium tel qu'une silice fonctionnalisée, une silicone ou un caoutchouc siliconé ; ou des oxydes inorganiques poreux et des chlorures inorganiques.

17. Procédé selon l'une quelconque des revendications précédentes, dans lequel la carbonylation est réalisée à une température comprise entre -10 et 150°C.

18. Procédé selon l'une quelconque des revendications précédentes, dans lequel la carbonylation est réalisée à une pression partielle de CO comprise entre 0,80 x 10⁵ N.m⁻² et 90 x 10⁵ N.m⁻².

19. Procédé selon l'une quelconque des revendications 1 à 17, dans lequel la carbonylation est réalisée à une faible pression partielle de CO comprise entre 0,1 et 5 x 10⁵ N.m⁻².

20. Procédé selon l'une quelconque des revendications précédentes, dans lequel la phosphine bidentée est choisie indépendamment parmi des composés quelconques suivants : le bis-(di-t-butylphosphino)-o-xylène (également connu sous le nom de 1,2-bis-(di-t-butylphosphinométhyl)benzène) ; le 1,2-bis-(diadamantylphosphinométhyl)benzène ; le 1,2-bis-(diadamantylphosphinométhyl)naphtalène ; le 1,2-bis-(di-t-pentylphosphino)-o-xylène (également connu sous le nom de 1,2-bis-(di-t-pentylphosphinométhyl)benzène) ; le bis-2,3-(di-t-butylphosphinométhyl)naphtalène ; le 1,2-bis(di-tert-butylphosphinométhyl)ferrocène ; le 1,2,3-tris-(di-tert-butylphosphinométhyl)ferrocène ; le 1,2-bis-(diadamantylphosphinométhyl)ferrocène ; et le 1,2-bis-(di-t-pentylphosphinométhyl)ferrocène.

21. Utilisation du système catalytique selon l'une quelconque des revendications précédentes pour la production d'un ester lactate ou de l'acide lactique de formule (II), ladite production comprenant les étapes de carbonylation d'un composé acétate de vinyle de formule (IV), suivie du traitement du produit ramifié (iso) de la carbonylation en vue de produire l'ester ou l'acide.

22. Utilisation d'un système catalytique tel que défini dans l'une quelconque des revendications 1 à 20, dans laquelle le catalyseur est fixé à un support.

23. Utilisation d'un catalyseur selon la revendication 21, dans laquelle le traitement est l'hydrolyse ou la trans-estérification.

24. Utilisation du catalyseur selon la revendication 23, dans laquelle le produit est hydrogéné après l'hydrolyse.
